# EUROPÄISCHE PATENTSCHRIFT

(11) **EP 3 843 966 B1**
(45) Veröffentlichungstag und Bekanntmachung des Hinweises auf die Patenterteilung: **05.10.2022**
(21) Anmeldenummer: 19762701.1
(22) Anmeldetag: 28.08.2019
(51) Int. Cl.: G01N 21/51, G01N 21/64, G01N 21/65, G01N 21/85, G01N 33/44, B29B 17/00, B29B 17/04, G01N 21/31, G01N 21/33, G01N 21/359, G01N 21/84

(54) **VERFAHREN UND VORRICHTUNG ZUM AUFBEREITEN, BEARBEITEN UND/ODER RECYCELN VON THERMOPLASTISCHEN KUNSTSTOFFMATERIALIEN**
METHOD AND DEVICE FOR TREATING, PROCESSING AND/OR RECYCLING OF THERMOPLASTIC MATERIALS
PROCÉDÉ ET DISPOSITIF POUR TRAITER, TRAVAILLER ET/OU RECYCLER DES MATÉRIAUX THERMOPLASTIQUES

(30) Priorität: 29.08.2018 AT 507382018
(43) Veröffentlichungstag der Anmeldung: 07.07.2021
(60) Teilanmeldung: 22192112.5
(73) Patentinhaber: EREMA Engineering Recycling Maschinen und Anlagen Gesellschaft m.b.H., 4052 Ansfelden (AT)
(72) Erfinder: HAIDER, Stephanie, 4030 Linz (AT); AIGNER, Michael, 4060 Leonding (AT); FEICHTINGER, Klaus, 4040 Linz (AT)
(74) Vertreter: Wildhack & Jellinek
(86) Internationale Anmeldenummer: PCT/AT2019/060278
(87) Internationale Veröffentlichungsnummer: WO 2020/041813

(56) Entgegenhaltungen:
- EP-A1- 0 123 771
- EP-A1- 2 689 908
- EP-A1- 3 537 119
- EP-A2- 1 264 170
- EP-A2- 3 128 303
- WO-A1-2017/051424

## Beschreibung

Verfahren und Vorrichtung zum Aufbereiten, Bearbeiten und/oder Recyceln von Materialien Die Erfindung betrifft ein Verfahren zum Aufbereiten, Bearbeiten bzw. Recyceln von Materialien, nämlich von thermoplastischem Kunststoffmaterial gemäß Patentanspruch 1. Die Erfindung betrifft weiters eine Vorrichtung zur Durchführung eines erfindungsgemäßen Verfahrens gemäß Patentanspruch 10.

Die Druckschrift EP 1 264 170 A2 offenbart ein Mehrkopfsondensystem für die spektroskopische Analyse eines sich bewegenden fließfähigen Materials und stellt ein Spektralanalysesystem mit einer Sonde bereit, die in einen sich schnell bewegenden Strom eingeführt werden kann, oder die durch ein Fenster einen sich in einem Behälter bewegenden Materialstrom mittels Bestrahlung mit ultraviolettem, sichtbarem oder infrarotem Licht analysieren kann. Dieses System kann prinzipiell in jedem körnigen Feststoff, jeder Flüssigkeit oder jedem Gas eingesetzt werden, das sich durch oder entlang eines geschlossenen oder offenen Kanals bewegt, wie beispielsweise Gülle, Erde, Schlamm, Bergbaumaterialien, Chemikalien, Arzneimittel, Lebensmittel, Abfallstoffe, gefährliche Abfälle, Erdöl und Erdölprodukte, Industriegase, Abgase usw.

Die Druckschrift EP 3 128 303 A2 offenbart die Überwachung von Herstellungsprozessen von pharmazeutischen Produkten, Lebensmitteln, Destillaten und anderen chemischen Verbindungen unter Verwendung eines Spektrometers.

Insbesondere beim Kunststoffrecycling ist es eine Hauptaufgabe, Kunststoffe, die aus unterschiedlichen Quellen mit unter Umständen unterschiedlicher Zusammensetzung, die auch unbekannt sein kann, aufzuarbeiten. Der Bereich des Kunststoffrecycelns umfasst dabei beispielsweise sowohl den Bereich der internen Produktionsabfälle als auch den Bereich der verwendeten Kunststoffprodukte, wie Verpackungen, Computergehäuse, oder Teile aus dem Automotive-Bereich usw., sodass die Ausgangsstoffe z.B. stark variierende Füllstoff- und Polymergehalte aufweisen.

Ziel der Aufbereitung ist es, für den Wiedereinsatz des Materials definierte Qualitätsmerkmale wie z.B. definierte mechanische, optische und/oder andere Eigenschaften zu erreichen. Damit diese Qualitätsmerkmale erzielt werden können, ist neben der nötigen maschinentechnischen Voraussetzung eine Analyse der eingehenden aber auch der verarbeiteten Materialien nötig.

Aufgabe der vorliegenden Erfindung ist es, ein Verfahren und eine Vorrichtung zum Aufbereiten, Bearbeiten und/oder Recyceln von Materialien wie thermoplastischen Kunststoffen bereitzustellen, mit der auf einfache und effektive Weise eine Aufbereitung von Materialen mit gleichzeitiger Möglichkeit zur Überwachung und Steuerung des Aufbereitungsprozesses zur Erzielung erwünschter Qualitätsmerkmale beim Prozessendprodukt bereitzustellen.

Die vorliegende Erfindung löst diese Aufgabe durch ein Verfahren gemäß den Merkmalen des Anspruchs 1. Erfindungsgemäß ist dabei vorgesehen, - dass das Material in einem Aufnahmebehälter, nämlich einem Schneidverdichter, bewegt und gemischt wird, und auch erwärmt, zerkleinert und/oder erweicht wird, wobei das Material im Aufnahmebehälter durchwegs stückig bzw. teilchenförmig und unaufgeschmolzen bleibt, und - dass das sich im Inneren des Aufnahmebehälters bewegende stückige bzw. teilchenförmige Material inline spektroskopisch und/oder spektrometrisch analysiert oder gemessen wird, wobei die derart ermittelten Messwerte zur Gewinnung von Informationen über das jeweils vermessene Material, insbesondere von quantitativen und/oder qualitativen Kenngrößen des jeweiligen Materials, herangezogen werden.

Durch diese Ausgestaltung eines erfindungsgemäßen Verfahrens ist es möglich, das sich im Aufnahmebehälter bewegende Material inline spektroskopisch und/oder spektrometrisch zu untersuchen und auf diese Weise Informationen über das Material, wie beispielsweise Additiv- oder Füllstoffgehalt und Polymerzusammensetzung, zu gewinnen, sodass der Verarbeitungsprozess entsprechend den Informationen, beispielsweise durch Zugabe von Füllstoffen oder Polymeren, gesteuert werden kann. Auf diese Weise sind beim Prozessendprodukt gewünschte Qualitätsmerkmale bzw. Eigenschaften genau einstellbar. Unter "inline" wird vorliegend verstanden, dass die Analyse bzw. die Vermessung des Materials im Aufnahmebehälter in die Aufbereitungs- bzw. Bearbeitungsprozess-Linie eingebunden ist und während der Aufbereitung und Bearbeitung des Materials direkt in der Linie erfolgt.

Die vorliegende Erfindung bezieht sich auf thermoplastisches Kunststoffmaterial. Eine besonders exakte Untersuchung des sich im Aufnahmebehälter befindlichen Materials kann erzielt werden, wenn zumindest Teile des im Inneren des Aufnahmebehälters befindlichen und dort rotierenden stückigen bzw. teilchenförmigen Materials durch eine physikalische Einwirkung, nämlich durch elektromagnetische Strahlung, angeregt wird und die als Reaktion auf die Einwirkung entstehenden Messsignale, insbesondere charakteristische Spektren der am vermessenen Material gestreuten elektromagnetischen Strahlung, vorzugsweise spektrometrisch, detektiert werden.

Um bei der Vermessung bzw. Analyse des sich im Aufnahmebehälter befindlichen Materials eine Vielzahl verschiedener physikalischer Prozesse zur Identifizierung von chemischen Substanzen nutzen zu können, ist erfindungsgemäß vorgesehen, dass die spektroskopische und/oder spektrometrische Vermessung mittels Atomspektroskopie oder Molekülspektroskopie erfolgt. Dabei kann es sich beispielsweise um Raman-Spektroskopie, NIR-Spektroskopie, UV/VIS-Spektroskopie, Fluoreszenz-Spektroskopie und/oder Absorptions-Spektroskopie handeln.

Um eine besonders gezielte Nutzung beispielsweise des RAMAN-Effekts, bei dem Energie vom Licht auf die Materie und umgekehrt übertragen wird, zu gewährleisten, ist erfindungsgemäß vorgesehen, dass zur Anregung Licht im Bereich des infraroten, des sichtbaren und/oder des UV-Lichts verwendet wird. Dabei wird erfindungsgemäß Licht mit einer Wellenlänge im Bereich von 100 nm bis 1400 nm, vorzugsweise von 500 bis 1000 nm verwendet, oder alternativ dazu ist eine Anregung mittels eines Lasers, mit einem Wellenlängenbereich von 100 nm bis 1400 nm und bevorzugt mit einer Leistung im Bereich von 15 mW bis 5 W, vorzugsweise von 100 bis 500 mW möglich.

Eine besonders einfache und gezielte Steuerung von physikalischen Eigenschaften bzw. Qualitätsmerkmalen beim Prozessendprodukt kann erzielt werden, wenn das detektierte Licht analysiert wird, um spezifische quantitative und/oder qualitative Kenngrößen des thermoplastischen Kunststoffmaterials, bzw. Veränderungen dieser Kenngrößen während des Prozesses inline zu erfassen. Diese Informationen bzw. Kenngrößen werden erfindungsgemäß zur Überwachung und/oder Steuerung des Prozesses herangezogen und/oder zur Steuerung der Prozessführung im Aufnahmebehälter verwendet. Das bedeutet, dass eine unmittelbare Reaktion auf Veränderungen der Kenngrößen während der Aufbereitung des Materials beispielsweise durch Zugabe von Füllstoffen möglich ist, sodass die Eigenschaften des Prozessendprodukts gezielt gesteuert werden können.

Um die detektierten Messsignale in besonders guter Qualität, d.h. mit geringem Hintergrundrauschen, messen zu können und die, die anregende elektromagnetische Strahlung abgebende, Anregungsquelle möglichst klein halten zu können, ist erfindungsgemäß vorgesehen, dass die das Material anregende elektromagnetische Strahlung auf einen Fokuspunkt fokussiert wird, der im Inneren des Aufnahmebehälters am oder unmittelbar hinter der Behälterwand, in einem Abstand von maximal 10 cm hinter der Behälterwand, liegt.

Um bei der Detektion der Messsignale besonders zuverlässig sicherzustellen, dass sie nicht durch oberflächliche Verschmutzung oder Beschichtung der zu vermessenden Materialteilchen beeinflusst werden, ist erfindungsgemäß vorgesehen, dass durch die physikalische Einwirkung, insbesondere die elektromagnetische Strahlung, ein durch eine Messfleck-Querschnittsfläche von 0,1 mm bis 5 mm, insbesondere von 1 mm bis 3 mm, und eine Eindringtiefe in das Material von 0,3 µm bis 30 µm, insbesondere von 8 µm bis 15 µm, definierter Volumenbereich angeregt wird.

Auf diese Weise können einzelne Teilchen untersucht werden, wobei die Anregung bis in tief liegende Bereiche des jeweiligen Teilchens reicht, die nicht mit z.B. Farbe überzogen sind, sodass ein repräsentativer Messwert für das Teilchen gewonnen werden kann.

Um besonders zuverlässig zu gewährleisten, dass ausreichend zu vermessendes Material von der physikalischen Einwirkung angeregt wird und keine Beeinflussung der Messung durch Fremdlicht erfolgt, kann vorgesehen sein, dass die das Material anregende physikalische Einwirkung, insbesondere die elektromagnetische Strahlung, an einer oder mehreren der folgenden Positionen in das Innere des Aufnahmebehälters bzw. in das Material eingebracht wird:
- unterhalb des Füllniveaus des im Betrieb im Aufnahmebehälter befindlichen Materials bzw. der Materialteilchen,
- in einer Höhe und/oder in einem Abstand zum Boden oder zum Misch- und/oder Zerkleinerungswerkzeug, in der/dem die physikalische Einwirkung, insbesondere die elektromagnetische Strahlung, ständig unterhalb des verfahrensmäßig vorgegebenen Füllstandes der im Aufnahmebehälter befindlichen bzw. rotierenden Materialteilchen und/oder unterhalb des Niveaus der bei einer Bewegung und/oder Rotation der Materialteilchen ausgebildeten Mischtrombe liegt,
- in Höhe des mittleren Drittelbereichs des verfahrensmäßig vorgegebenen Füllstandes des Materials im Aufnahmebehälter und/oder der Mischtrombe,
- in demjenigen Bereich des Aufnahmebehälters, in dem die Dichte der bewegten und/oder rotierenden Materialteilchen am höchsten ist, und/oder
- in demjenigen Bereich des Aufnahmebehälters, in dem die bewegten und/oder rotierenden Materialteilchen den höchsten Druck auf die Seitenwand des Aufnahmebehälters ausüben.

Ein besonders häufiger und regelmäßiger Austausch des Materials an der Messposition bzw. im Fokuspunkt wird erfindungsgemäß dadurch erzielt, dass das stückige bzw. teilchenförmige Material im äußeren Bereich des Aufnahmebehälters, insbesondere an der Seitenwand des Aufnahmebehälters, eine Bewegungsrichtung in Umfangsrichtung und eine vorwiegend aufwärtsgerichtete Bewegungsrichtung aufweist.

Eine weitere Verbesserung des Austauschs von Material an der Messposition bzw. im Fokuspunkt wird erfindungsgemäß dadurch gewährleistet, dass das stückige bzw. teilchenförmige Material radial mit einer Geschwindigkeit von 0,3 m/s bis 45 m/s und in vertikaler Richtung mit einer Geschwindigkeit von 0,1 m/s bis 60 m/s umläuft. Auf diese Weise kann erzielt werden, dass das stückige bzw. teilchenförmige Material im äußeren Bereich des Aufnahmebehälters, insbesondere an der Seitenwand des Aufnahmebehälters, häufig und regelmäßig ausgetauscht wird.

Eine besonders exakte Möglichkeit zur Ableitung von Informationen über das sich im Inneren des Aufnahmebehälters befindliche Material kann bereitgestellt werden, wenn das im Inneren des Aufnahmebehälters befindliche und dort rotierende stückige bzw. teilchenförmige Material, insbesondere einzelne Teilchen des Materials, zu einer Vielzahl an vorgegebenen Zeitpunkten durch eine physikalische Einwirkung, nämlich elektromagnetische Strahlung, angeregt wird und der Mittelwert der Informationen über das jeweils vermessene Material, insbesondere über die einzelnen Teilchen, ermittelt und zur Verfügung gehalten wird. Dabei kann es sich vorzugweise um den Mittelwert der quantitativen und/oder qualitativen Kenngrößen des jeweiligen Materials bzw. der jeweiligen Teilchen, die auf Grundlage ausgewählter, vorzugsweise aller, zu diesen Zeitpunkten ermittelten Messwerte ermittelt wurden, handeln. Somit ergibt sich ein Mittelwert aus den Messwerten der einzelnen vermessenen Teilchen.

Eine weitere besonders genaue Möglichkeit zur Gewinnung von Informationen über das sich im Inneren des Aufnahmebehälters befindliche Material, bei der gleichzeitig auch eine physikalische Einwirkung, die von einer Anregungsquelle mit besonders geringer Leistung abgegeben wird, ausreicht, kann bereitgestellt werden, wenn das im Inneren des Aufnahmebehälters befindliche und dort rotierende stückige bzw. teilchenförmige Material während eines vorgegebenen Zeitraums, insbesondere von mehreren Sekunden, kontinuierlich durch eine physikalische Einwirkung, nämlich elektromagnetische Strahlung, angeregt wird. Für den jeweiligen Zeitraum wird eine gemeinsame Information über das jeweils vermessene Material, insbesondere eine quantitative und/oder qualitative Kenngröße, auf Grundlage der innerhalb dieses Zeitraums kontinuierlich ermittelten Messwerte berechnet und zur Verfügung gehalten. Somit ergibt sich ein kumulativer Messwert für alle Teilchen, die sich im Messzeitraum an der Messposition bzw. dem Fokuspunkt vorbeibewegt haben.

Um durch Temperaturänderungen bedingte Effekte, die die vom zu vermessenden Material ausgehenden Messsignale bzw. die damit verbundenen Informationen über das Material verfälschen könnten, besonders effektiv korrigieren zu können, kann vorgesehen sein, dass die Temperatur im Inneren des Aufnahmebehälters und/oder die Temperatur des Materials gemessen wird und die Temperaturinformation in die Auswertung einbezogen wird. Die gemessene Temperaturinformation kann als Indikation für die Korrektur der Informationen über das jeweils vermessene Material, insbesondere der quantitativen und/oder qualitativen Kenngrößen des jeweiligen Materials, dienen. Zusätzlich oder alternativ dazu kann die Materialtemperatur erfasst werden und als Vorgabe für die Korrektur der Spektren herangezogen werden.

Eine besonders exakte Auswertung der für das Material ermittelten Informationen kann gewährleistet werden, wenn Referenzinformationen, insbesondere quantitative und/oder qualitative Referenzkenngrößen, vorzugsweise Referenzspektren, hinterlegt werden und die für das jeweils vermessene Material ermittelten Informationen, insbesondere die Kenngrößen, vorzugsweise die Spektren, mit den Referenzinformationen, insbesondere den Referenzkenngrößen, vorzugsweise den Referenzspektren, verglichen werden. Die Abweichung von den Referenzinformationen, insbesondere den Referenzkenngrößen, vorzugsweise den Referenzspektren, kann so besonders einfach bestimmt, und insbesondere angezeigt und/oder zur Überwachung und/oder Steuerung der Prozessführung im Aufnahmebehälter und/oder der nachfolgenden Prozesskette herangezogen werden.

Aufgabe der Erfindung ist es weiters, eine Vorrichtung zur Durchführung eines erfindungsgemäßen Verfahrens zum Aufbereiten, Bearbeiten und/oder Recyceln von thermoplastischen Kunststoffmaterialien, zur Verfügung zu stellen. Die Erfindung löst diese Aufgabe mit einer Vorrichtung mit den Merkmalen gemäß Patentanspruch 10.

Erfindungsgemäß ist dabei vorgesehen, dass die Vorrichtung zumindest einen Aufnahmebehälter, nämlich einen Schneidverdichter, mit einer Misch- und/oder Zerkleinerungseinrichtung für das Material sowie mit einer spektroskopischen und/oder spektrometrischen Messvorrichtung zur Analyse des sich im Inneren des Aufnahmebehälters bewegenden stückigen bzw. teilchenförmigen Materials bzw. zur Gewinnung von Informationen über das jeweils vermessene thermoplastische Kunststoffmaterial, insbesondere von quantitativen und/oder qualitativen Kenngrößen des jeweiligen thermoplastischen Kunststoffmaterials, umfasst.

Durch diese Ausgestaltung einer erfindungsgemäßen Vorrichtung ist es möglich, dass inline eine spektroskopische und/oder spektrometrische Vermessung bzw. Analyse des sich im Aufnahmebehälter bewegenden thermoplastischen Kunststoffmaterials vorzunehmen. So können Informationen über das Material, wie beispielsweise Additiv- oder Füllstoffgehalt und Polymerzusammensetzung inline ermittelt werden, sodass der Verarbeitungsprozess entsprechend den Informationen gesteuert werden kann, um beim Prozessendprodukt gewünschte Qualitätsmerkmale bzw. Eigenschaften genau einzustellen, beispielsweise durch Zugabe von Füllstoffen oder Polymeren.

Eine besonders kompakte Ausführungsform einer erfindungsgemäßen Vorrichtung, die inline eine Vermessung und Analyse des zu verarbeitenden Materials ermöglicht, wird dadurch bereitgestellt, dass diese zumindest einen Aufnahmebehälter, nämlich einen Schneidverdichter, mit einer Misch- und/oder Zerkleinerungseinrichtung für das Material, zumindest eine spektroskopische und/oder spektrometrische Messvorrichtung zur Inline-Vermessung von Teilen des sich im Inneren des Aufnahmebehälters bewegenden stückigen bzw. teilchenförmigen Materials, und eine mit der Messvorrichtung in Datenkommunikation stehende Verarbeitungs- und Steuereinheit umfasst.

Die Messvorrichtung ist erfindungsgemäß dazu ausgebildet, eine physikalische Einwirkung, nämlich elektromagnetische Strahlung, zur Anregung des rotierenden stückigen bzw. teilchenförmigen Materials abzugeben und die als Reaktion auf die Einwirkung entstehenden Messsignale, insbesondere charakteristische Spektren der am vermessenen Material gestreuten elektromagnetischen Strahlung, vorzugsweise spektrometrisch, zu detektieren.

Die Verarbeitungs- und Steuereinheit ist erfindungsgemäß dazu ausgebildet, die Messvorrichtung anzusteuern, die physikalische Einwirkung, nämlich elektromagnetische Strahlung, abzugeben und die entstehenden Messsignale zu detektieren und die derart ermittelten Messwerte zur Verfügung zu halten und gegebenenfalls auf Grundlage der ermittelten Messwerte Informationen über das jeweils vermessene Material, insbesondere quantitative und/oder qualitative Kenngrößen des jeweiligen Materials, abzuleiten und zur Verfügung zu halten.

Ein für die Aufbereitung, Bearbeitung oder das Recycling von Materialien besonders geeigneter Aufnahmebehälter wird erfindungsgemäß dadurch bereitgestellt, dass der Aufnahmebehälter eine Seitenwand besitzt und im Wesentlichen konisch oder zylindrisch ist oder einen konischen oder zylindrischen Wandabschnitt aufweist, und gegebenenfalls auch eine untere Bodenfläche aufweist.

Eine besonders effiziente Aufbereitung von verschiedensten Materialien wird erfindungsgemäß dadurch gewährleistet, dass im Aufnahmebehälter als Misch- und/oder Zerkleinerungseinrichtung zumindest ein, insbesondere um eine vertikale Drehachse drehbares, umlaufendes Misch- und/oder Zerkleinerungswerkzeug zur Bewegung und Mischung, und gegebenenfalls auch zur Erwärmung, Zerkleinerung und/oder Erweichung, des zu behandelnden stückigen bzw. teilchenförmigen Materials angeordnet ist, wobei im Betrieb im Aufnahmebehälter ein Wirbel und/oder eine Mischtrombe, ausbildbar ist.

Bei nicht erfindungsgemäßen Anwendungen, bei denen eine zu starke Durchmischung der Teilchen unerwünscht ist, kann derart vorteilhafterweise erreicht werden, dass das Material einen Wirbel ausbildet und weitgehend nur in einer Ebene umläuft, während entlang der Längsachse des Aufnahmebehälters der Materialdurchlauf beispielsweise mit einer Geschwindigkeit von 2 m/h mit einer Umfangsgeschwindigkeit von beispielsweise ca. 0,3 m/s erfolgt. Bei Anwendungen, bei denen eine besonders gute Durchmischung erwünscht ist, kann eine Mischtrombe ausgebildet werden.

Eine besonders effektive Aufbereitung der im Aufnahmebehälter befindlichen Materialien wird erfindungsgemäß dadurch erzielt, dass die Umfangsgeschwindigkeit des Misch- und/oder Zerkleinerungswerkzeugs derart gewählt ist, dass das stückige bzw. teilchenförmige Material radial mit einer Geschwindigkeit von 0,3 m/s bis 45 m/s und/oder in vertikaler Richtung mit einer Geschwindigkeit von 0,1 m/s bis 60 m/s umläuft. So ist vorteilhafterweise auch sichergestellt, dass das Material an der Messposition bzw. dem Fokuspunkt ständig ausgetauscht wird.

Ein besonders einfaches Ausbringen von aufbereiteten Materialien aus dem Aufnahmebehälter kann gewährleistet werden, wenn im Aufnahmebehälter, insbesondere in einer Seitenwand des Aufnahmebehälters, eine Öffnung ausgebildet ist, durch die das vorbehandelte Kunststoffmaterial aus dem Inneren des Aufnahmebehälters ausbringbar ist und wenn zumindest ein Förderer, insbesondere ein Extruder, mit zumindest einer in einem Gehäuse rotierenden, insbesondere plastifizierenden oder agglomerierenden, Schnecke, zur Aufnahme des aus der Öffnung austretenden vorbehandelten Materials angeordnet ist.

Für eine weitere Vereinfachung der Ausbringung kann sich die Öffnung im Aufnahmebehälter im Bereich der Höhe des oder des untersten, bodennächsten Misch- und/oder Zerkleinerungswerkzeugs befinden und das Gehäuse kann eine an seiner Stirnseite oder in seiner Mantelwand liegende Einzugsöffnung für das von der Schnecke zu erfassende Material aufweisen, die mit der Öffnung in Verbindung steht.

Eine besonders kompakte Ausführungsform der Messvorrichtung einer erfindungsgemäßen Vorrichtung wird dadurch bereitgestellt, dass die Messvorrichtung zumindest eine, ins Innere des Aufnahmebehälters wirkende bzw. gerichtete Anregungsquelle zur Abgabe einer physikalischen Einwirkung, nämlich von elektromagnetischer Strahlung, auf zumindest Teile des im Betrieb im Inneren des Aufnahmebehälters befindlichen und dort rotierenden stückigen bzw. teilchenförmigen Materials sowie zumindest einen Detektor, insbesondere ein Spektroskop, zur Erfassung der als Reaktion auf die Einwirkung entstehenden Messsignale, insbesondere charakteristischer Spektren der am vermessenen Material gestreuten elektromagnetischen Strahlung, umfasst.

Die Anregungsquelle kann dabei z.B. von Richtung der Behälterwand des Aufnahmebehälters aus oder ausgehend vom Misch- und/oder Zerkleinerungswerkzeug aus oder von der Bodenfläche des Aufnahmebehälters her ins Innere des Aufnahmebehälters wirken.

Eine besonders zuverlässige Charakterisierung der im Aufnahmebehälter befindlichen Materialteilchen wird erfindungsgemäß dadurch erzielt, dass die Verarbeitungs- und Steuereinheit zur spektrometrischen und/oder spektroskopischen Analyse der als Reaktion auf die Einwirkung entstehenden Messsignale, insbesondere von charakteristischen Spektren der am vermessenen Material gestreuten elektromagnetischen Strahlung, ausgebildet ist.

Eine besonders einfache Möglichkeit, vom Aufnahmebehälter ausgehende, störende Einflüsse auf die Anregungsquelle und/oder den Detektor der Messvorrichtung einer erfindungsgemäßen Vorrichtung zu vermeiden, kann bereitgestellt werden, wenn die Anregungsquelle und/oder der Detektor und/oder die Verarbeitungs- und Steuereinheit vom Aufnahmebehälter physikalisch beabstandet oder am Aufnahmebehälter vibrationsfrei, insbesondere über faseroptische Systeme und/oder Lichtleiter, angekoppelt sind.

Um verschiedenste physikalische Prozesse zur Anregung und für die chemische Analyse des sich im Aufnahmebehälter befindlichen Materials ausnutzen zu können, kann vorgesehen sein, dass die Messvorrichtung eine Vorrichtung, insbesondere einen Detektor, zur Atomspektroskopie oder Molekülspektroskopie, insbesondere zur Raman-Spektroskopie, NIR-Spektroskopie, UV/VIS-Spektroskopie, Fluoreszenz-Spektroskopie und/oder Absorptions-Spektroskopie, umfasst.

Um das Material im Inneren des Aufnahmebehälters anhand seiner Reaktion auf Licht in unterschiedlichen Wellenlängenbereichen analysieren bzw. charakterisieren zu können, ist erfindungsgemäß vorgesehen, dass die Anregungsquelle dazu ausgebildet ist, Licht im Bereich des infraroten, des sichtbaren und/oder des UV-Lichts, insbesondere im Bereich von 100 nm bis 1400 nm, vorzugsweise von 500 nm bis 1050 nm, abzugeben.

Eine besonders vielseitig einsetzbare Anregungsquelle wird erfindungsgemäß bereitgestellt, wenn die Anregungsquelle ein Laser ist, mit einem Wellenlängenbereich von 100 nm bis 1400 nm oder einer Leistung im Bereich von 15 mW bis 5 W, vorzugsweise von 100 mW bis 500 mW.

Eine besonders zuverlässige Vermessung der Materialien im Inneren des Aufnahmebehälters bei gleichzeitiger physischer Trennung des Aufnahmebehälters von der Messvorrichtung, sodass die Messvorrichtung weitgehend von störenden Einflüssen, die vom Aufnahmebehälter ausgehen, unbeeinflusst bleibt, wird erfindungsgemäß dadurch bereitgestellt, dass im Aufnahmebehälter, insbesondere in der Seitenwand des Aufnahmebehälters, zumindest eine Messöffnung vorgesehen ist. Dabei ist die Messöffnung derart ausgebildet, dass die von der Anregungsquelle abgegebene physikalische Einwirkung, insbesondere die abgegebene elektromagnetische Strahlung, auf das Material im Aufnahmebehälter einwirkt und Streulicht aus dem Aufnahmebehälter außerhalb des Aufnahmebehälters detektierbar ist.

Eine Messöffnung, die besonders geringe konstruktive Änderungen am Aufnahmebehälter, die eine statische Schwächung des Aufnahmebehälters bewirken könnten, bedingt, kann bereitgestellt werden, wenn die Messöffnung einen Durchmesser von 0,5 mm bis 100 mm hat.

Um die vom Material im Inneren des Aufnahmebehälters ausgehenden Messsignale weitestgehend unverfälscht aufnehmen zu können, kann vorgesehen sein, dass die Messöffnung durch ein Fenster aus für elektromagnetische Strahlung, durchlässigem Material, beispielsweise aus Saphirglas, verschlossen ist. Um eine möglichst geringe Verfälschung der vom Material im Inneren des Aufnahmebehälters ausgehenden Messsignale zu gewährleisten, kann weiters vorgesehen sein, dass das Fenster eine Stärke von 1 mm bis 100 mm aufweist.

Um die Stabilität des Aufnahmebehälters durch die Anordnung eines Fensters bzw. einer Messöffnung, die durch ein Fenster verschlossen ist, möglichst wenig zu beeinflussen, kann vorgesehen sein, dass die Flächen des Fensters planeben und zueinander parallel ausgerichtet sind. Alternativ kann dieser Effekt erzielt werden, wenn die dem Aufnahmebehälter zugewendete inneren Fläche des Fensters konkav dem Radius des Aufnahmebehälters angepasst ist und die dem Aufnahmebehälter abgewendete äußere Fläche des Fensters parallel konkav zur inneren Fläche ausgebildet ist.

Um besonders zuverlässig zu erzielen, dass ausreichend Material an der Messöffnung vorbeitransportiert wird, ist erfindungsgemäß vorgesehen, dass die zumindest eine Messöffnung, insbesondere in der Seitenwand des Aufnahmebehälters, an einer oder mehreren der folgenden Positionen angeordnet ist/sind:
- im Bereich der Höhe des untersten, bodennächsten Misch- und/oder Zerkleinerungswerkzeugs, insbesondere etwas oberhalb oder unterhalb davon, vorzugsweise beim engsten Abstand zwischen dem äußersten Punkt des Misch- und/oder Zerkleinerungswerkzeugs und der Seitenwand, und/oder
- im Bereich des unteren Drittels der Höhe des Aufnahmebehälters.

Eine besonders vorteilhafte Positionierung der Messöffnung, die eine besonders genaue Analyse bzw. Vermessung des im Inneren des Aufnahmebehälters befindlichen Materials gewährleistet, kann erzielt werden, wenn die zumindest eine Messöffnung, insbesondere in der Seitenwand des Aufnahmebehälters, an einer oder mehreren der folgenden Positionen angeordnet ist/sind:
- unterhalb des Füllniveaus des im Betrieb im Aufnahmebehälter befindlichen Materials bzw. der Materialteilchen,
- in einer Höhe und/oder in einem Abstand zum Boden oder zum Misch- und/oder Zerkleinerungswerkzeug, in der/dem die physikalische Einwirkung, insbesondere die elektromagnetische Strahlung, ständig unterhalb des verfahrensmäßig vorgegebenen Füllstandes der im Aufnahmebehälter befindlichen bzw. rotierenden Materialteilchen und/oder des Niveaus der bei einer Bewegung und/oder Rotation der Materialteilchen ausgebildeten Mischtrombe liegt,
- in Höhe des mittleren Drittelbereichs des verfahrensmäßig vorgegebenen Füllstandes des Materials im Aufnahmebehälter und/oder der Mischtrombe,
- in demjenigen Bereich des Aufnahmebehälters, in dem die Dichte der bewegten und/oder rotierenden Materialteilchen am höchsten ist, und/oder
- in demjenigen Bereich des Aufnahmebehälters, in dem die bewegten und/oder rotierenden Materialteilchen den höchsten Druck auf die Seitenwand des Aufnahmebehälters ausüben.

An allen diesen Positionen ist sichergestellt, dass sich ausreichend häufig wechselndes Material an der Messöffnung vorbeibewegt und keine Beeinflussung der Messung durch Fremdlicht erfolgt.

Eine besonders geringe Beeinflussung der vom vermessenen Material ausgehenden Messsignale durch beispielsweise Beschichtungen oder Verschmutzungen an der Oberfläche der Materialteilchen wird erfindungsgemäß dadurch erzielt, dass der durch die physikalische Einwirkung, insbesondere die elektromagnetische Strahlung, anregbare Volumenbereich durch eine Messfleck-Querschnittsfläche von 0,1 mm bis 5 mm, insbesondere von 1 mm bis 3 mm, und eine Eindringtiefe in das Material von 0,3 µm bis 30 µm, insbesondere von 8 µm bis 15 µm, definiert ist.

Ein besonders günstiges Signal-Rausch-Verhältnis der Messsignale wird erfindungsgemäß dadurch erzielt, dass eine Linse oder ein Linsensystem zur Fokussierung der elektromagnetischen Strahlung der Anregungsquelle auf einen Fokuspunkt vorgesehen ist, wobei der Fokuspunkt insbesondere am oder unmittelbar hinter dem Fenster, vorzugsweise in einem Abstand von maximal 10 cm hinter dem Fenster, ausgebildet ist.

Eine besonders gezielte Steuerung des Aufbereitungs- bzw. Recyclingprozesses der thermoplastischen Kunststoffmaterialien, wird erfindungsgemäß dadurch erzielt, dass Verarbeitungs- und Steuereinheit mit einer Prozess-Steuerungseinheit zusammenwirkt und in Datenkommunikation steht. Eine derartige Prozess-Steuerungseinheit ist dazu ausgebildet, von der Verarbeitungs- und Steuereinheit übermittelte Daten, insbesondere Informationen über das jeweils vermessene thermoplastische Kunststoffmaterial, vorzugsweise quantitative und/oder qualitative Kenngrößen des jeweiligen thermoplastischen Kunststoffmaterials, zur Überwachung und/oder Steuerung der Prozessführung im Aufnahmebehälter und/oder der nachfolgenden Prozesskette zu nutzen.

Eine besonders effektive Steuerung der Qualitätsmerkmale bzw. der gewünschten beispielsweise optischen oder physikalischen Eigenschaften des Prozessendprodukts kann gewährleistet werden, wenn die Prozess-Steuerungseinheit dazu ausgebildet ist, auf Grundlage der von der Verarbeitungs- und Steuereinheit übermittelten Daten
- eine Zudosierung von Füllstoffen in den Aufnahmebehälter vorzunehmen und/oder
- eine Zuführung von Materialien, insbesondere Polymeren, in den Aufnahmebehälter und/oder in eine an den Aufnahmebehälter angeschlossene Austragungsvorrichtung vorzunehmen und/oder
- verarbeitete Materialien, insbesondere Granulat, aus dem Aufnahmebehälter mittels der an den Aufnahmebehälter angeschlossenen Austragungsvorrichtung auszuschleusen.

Eine besonders einfache Berücksichtigung von Temperatureinflüssen, die die vom zu vermessenden Material abgegebenen Messsignale beeinflussen können, kann bereitgestellt werden, wenn zumindest eine der Verarbeitungs- und Steuereinheit vorgeschaltete Temperaturmesseinrichtung vorgesehen ist. Eine derartige Temperaturmesseinrichtung ist dazu ausgebildet, die Temperatur im Inneren des Aufnahmebehälters und/oder die Temperatur des Materials zu messen und an die Verarbeitungs- und Steuereinheit zu übermitteln. Die Verarbeitungs- und Steuereinheit ist in diesem Fall vorteilhafterweise dazu ausgebildet ist, die von der zumindest einen Temperaturmesseinrichtung ermittelten Messwerte für eine Korrektur des Temperatureinflusses auf die für das jeweils vermessene Material ermittelten Informationen, insbesondere der temperaturabhängigen charakteristischen Spektren der am vermessenen Material gestreuten elektromagnetischen Strahlung, heranzuziehen und die derart korrigierten Informationen, insbesondere Spektren, zur Verfügung zu halten.

Eine besonders exakte Korrektur von Temperatureinflüssen kann erzielt werden, wenn die Temperaturmesseinrichtung im Aufnahmebehälter auf gleicher Höhe, insbesondere an gleicher Position, wie die zumindest eine Messöffnung angeordnet ist.

Eine Möglichkeit, besonders zuverlässige Informationen über das im Inneren des Aufnahmebehälters befindliche Material zu gewinnen, kann bereitgestellt werden, wenn die Verarbeitungs- und Steuereinheit dazu ausgebildet ist,
- die Messvorrichtung, insbesondere die Anregungsquelle, zu einer Vielzahl an vorgegebenen Zeitpunkten anzusteuern, wiederholt eine physikalische Einwirkung, nämlich elektromagnetische Strahlung, abzugeben und
- den Mittelwert der Informationen über das jeweils vermessene Material, insbesondere über die einzelnen vermessenen Teilchen, vorzugweise der quantitativen und/oder qualitativen Kenngrößen des jeweiligen Materials, die auf Grundlage ausgewählter, vorzugsweise aller, zu diesen Zeitpunkten von der Messvorrichtung, insbesondere dem Detektor, ermittelten Messwerte ermittelt wurden, zu berechnen und zur Verfügung zu halten.

Eine weitere Möglichkeit, besonders zuverlässige Informationen über das Material im Inneren des Aufnahmebehälters abzuleiten, bei der vorteilhafterweise eine Anregungsquelle mit besonders geringer Leistung ausreicht, kann bereitgestellt werden, wenn die Verarbeitungs- und Steuereinheit dazu ausgebildet ist,
- die Messvorrichtung, nämlich die Anregungsquelle, anzusteuern, eine physikalische Einwirkung, nämlich elektromagnetische Strahlung, während eines vorgegebenen Zeitraums, insbesondere von mehreren Sekunden, kontinuierlich abzugeben und
- für den jeweiligen Zeitraum eine gemeinsame Information über das jeweils vermessene Material, insbesondere eine quantitative und/oder qualitative Kenngröße, auf Grundlage der von der Messvorrichtung, nämlich dem Detektor, innerhalb dieses Zeitraums kontinuierlich ermittelten Messwerte zu berechnen und zur Verfügung zu halten.

Eine besonders einfache Auswertung bzw. Analyse der von der Verarbeitungs- und Steuereinheit ermittelten Informationen wie beispielsweise Spektren kann bereitgestellt werden, wenn die Verarbeitungs- und Steuereinheit einen Speicher umfasst, wobei in dem Speicher Referenzinformationen, insbesondere quantitative und/oder qualitative Referenzkenngrößen, vorzugsweise Referenzspektren, hinterlegt sind und wenn die Verarbeitungs- und Steuereinheit dazu ausgebildet ist, die für das jeweils vermessene Material ermittelten Informationen, insbesondere die Kenngrößen, vorzugsweise die Spektren, mit den Referenzinformationen, insbesondere den Referenzkenngrößen, vorzugsweise den Referenzspektren, zu vergleichen und die Abweichung von den Referenzinformationen, insbesondere den Referenzkenngrößen, vorzugsweise den Referenzspektren, zu bestimmen, und insbesondere an die Prozess-Steuerungseinheit und/oder eine Anzeigeeinheit weiterzuleiten.

Vorliegend wird unter einer Messvorrichtung eine Vorrichtung zur Aufnahme bzw.

Darstellung und quantitativen und/oder qualitativen Analyse eines Spektrums verstanden, die eine Anregungsquelle und einen Detektor umfasst. Die Anregungsquelle und der Detektor sind dabei aufeinander abgestimmt. Bei einer derartigen Messvorrichtung kann es sich um ein Spektrometer handeln.

Vorliegend wird unter einem Detektor eine Vorrichtung zur Detektion und Zerlegung von Strahlung oder anderen physikalischen Messwerten in ein Spektrum verstanden, die in einer Messvorrichtung integriert sein kann. Bei einem derartigen Detektor kann es sich um ein Spektroskop handeln.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben.

Im Folgenden zeigen schematisch:
Fig. 1 ein Ausführungsbeispiel einer erfindungsgemäßen Vorrichtung zum Aufbereiten, Bearbeiten und/oder Recyceln von thermoplastischen Kunststoffmaterialien mit einem an einen Aufnahmebehälter angebundenen RAMAN-Spektrometer,
Fig. 2 zeigt ein Ausführungsbeispiel der Vorrichtung aus Fig. 1 mit einer Temperaturmesseinrichtung,
Fig. 3a, Fig. 3b und Fig. 4 zeigen weitere Darstellungen der Ankopplung einer Messvorrichtung an einen Aufnahmebehälter,
Fig. 5 und Fig. 6 zeigen Beispiele von Stokes- und Anti-Stokes-Linien bei unterschiedlichen Wellenlängen,
Fig. 7 zeigt aufgenommene Spektren von LPDE-Material mit unterschiedlichen Gehalten an CaCO₃ als Füllstoff,
Fig. 8 zeigt die aus den Spektren in Fig. 7 abgeleitet Schwankung des CaCO₃-Gehalts über die Prozessdauer,
Fig. 9 zeigt aufgenommene Spektren von für Mischungen aus PP- und PE-Materialien mit unterschiedlichen Zusammensetzungen,
Fig. 10 zeigt den anhand der Spektren aus Fig. 9 ermittelten PP- und PE-Gehalt für verschiedene Messzeitpunkte,
Fig. 11 zeigt den anhand von Spektren ermittelten PA- und PE-Gehalt für verschiedene Messzeitpunkte.

### Allgemeines

Ein wesentlicher Teil der Aufbereitung der thermoplastischen oder teilthermoplastischen Materialien wird bei Betrieb einer Schneidverdichter-Extruder-Kombination, d.h. eines Zusammenbaus eines Schneidverdichters, einer sog. "Preconditioning Unit" PCU, mit einem Extruder, vom Schneidverdichter übernommen. Ein derartiger Schneidverdichter wird im Folgenden als Aufnahmebehälter 1 bezeichnet.

Bei einem Aufnahmebehälter 1 handelt sich dabei um einen im Wesentlichen zylindrischen Behälter, der eine Misch- und/oder Zerkleinerungseinrichtung, d.h. z.B. Rühr-Schneid-Umwälzwerkzeuge, enthalten kann, die vornehmlich von unten bzw. von dem, einem Förderer, z.B. einem Extruder 5, nächsten Punkt, nach oben aufgebaut sind. Die Werkzeuge können sowohl schneidend, rührend, mischend oder als Kombinationen davon ausgebildet sein. Derartige Vorrichtungen sind zB aus der EP 2 689 908 B1 oder der EP 2 525 953 B1 oder der EP 0 123 771 A1 bekannt.

Das zu bearbeitende Material verhält sich im Wesentlichen wie ein Fluid. Es liegt entweder schon stückig vor oder es wird durch die Werkzeuge in solch eine stückige Form gebracht. Durch das Schneiden, Rühren und/oder Mischen wird Energie in das Material gebracht, und das Material wird gleichmäßig erwärmt, wobei unabhängig von der Dicke der Materialteilchen eine vollständige Durchwärmung erreicht werden soll. Durch die Erweichung, durch Rückbildung der Verstreckung kann sich die Schüttdichte erhöhen. Weiters wird durch die Erwärmung die Voraussetzung geschaffen, dass leichter flüchtige Stoffe entweichen können, die zum Teil unerwünscht sind. Weiters können sich je nach Typ des thermoplastischen Polymers Gefügeveränderungen, z.B. eine Veränderung der Kristallisation, ergeben.

Das fertig aufbereitete Material kann dann entweder kontinuierlich oder auch batchweise aus dem Aufnahmebehälter 1 ausgetragen werden. Als Austragevorrichtungen bzw. Förderer eignen sich Klappen, Förderschnecken oder Extruder-Schnecken, die so angebracht sind, dass das Material zumindest durch die reine Fliehkraft ausgetragen wird. Allerdings ist auch ein Zwangsaustrag durch Kippen oder Stürzen möglich. Weiters kann auch ein Förderwerkzeug in den Aufnahmebehälter 1 eingebracht werden und das vorbehandelte Material ausgebracht werden. Für den kontinuierlichen Betrieb ist die bevorzugte Variante eine kontinuierliche Austragung des Materials.

Physikalische Methoden, die die auf der Wechselwirkung zwischen einer physikalischen Einwirkung wie z.B. elektromagnetischer Strahlung und Materie beruhen und z.B. das Energiespektrum einer Probe anhand von Strahlung bzw. Wellen untersuchen, wie z.B. Frequenzspektren-Analyseverfahren wie NIR, RAMAN, MIR etc. werden schon lange im Kunststoffrecycling diskutiert. Unter derartigen physikalischen Verfahren sind im Zusammenhang mit der Erfindung Verfahren zur Identifikation von chemischen Substanzen durch physikalische Anregung, wie beispielsweise durch einen Laser, verstanden. Durch diese Anregung werden Prozesse wie Rotations-, Schwingungs-, Phonon- oder Spin-Flip angeregt und diese führen zu einer Veränderung der Ladungsträgerdichte, was zur Identifizierung von chemischen Substanzen genutzt werden kann.

Zum Teil werden derartige physikalische Methoden in Sortierprozessen im Kunststoffrecycling eingesetzt. Beispielsweise in optischen Sortiersystemen wird FT-NIR zur Trennung von unterschiedlichen Kunststoffarten eingesetzt. Im Wesentlichen liegt hier ein stückiges vereinzeltes Gut vor, dass nach dem gut/schlecht-Prinzip aussortiert wird. Im Labor bzw. zur Materialidentifikation als Handgeräte sind ähnliche Systeme im Einsatz.

In der Extrusionstechnik wurden derartige Verfahren bisher zur Schmelze- und Eigenschaftscharakterisierung nur versuchsweise von Inhaltsstoffen eingesetzt, da die statistischen Voraussetzungen, d.h. ein kleiner Messbereich, lange Messzeiten, geringe Homogenisierung bei Polymermischungen, geringe Eindringtiefe in die Schmelze, und die bei vielen Polymeren auftretende Wandhaftung, d.h. das Material wird nicht ausgetauscht, Aussagen vornehmlich nur für ein einziges Polymer vernünftig zulassen.

Im Unterschied zum Einsatz von spektroskopischen Messsystemen beim Sortieren von Rohflakes oder bei Polymerschmelzen in einem Extruder kamen in Schneidverdichter-Systemen, also bei heißen, erweichten, aber immer stückigen, nicht aufgeschmolzenen und zudem rasch im Behälter herumwirbelnden teilchenförmigen Material-Partikeln, bei sich zwangsläufig ändernder Temperatur, derartige spektroskopische Messungen zur Inline-Prozessüberwachung und gegebenenfalls -steuerung auf Grund der zuvor genannten Einschränkungen bisher noch nicht zum Einsatz.

Die Messsysteme der physikalischen Methoden, die auf der Wechselwirkung zwischen einer physikalischen Einwirkung wie z.B. elektromagnetischer Strahlung und Materie beruhen, und z.B. des Energiespektrums einer Probe anhand von Strahlung bzw. Wellen untersuchen, sind im Wesentlichen Volumenmesssysteme, die durch Anregung, z.B. durch Laser oder Infrarotlicht, vom Material reflektierte Spektren erfassen und entweder direkt auswerten oder gegen Bibliotheken abgleichen. Aus diesem Grund beeinflussen die Häufigkeit der Messungen und der erfasste "Volumenbereich" wie repräsentativ das erzielte Messergebnis ist.

Ein Beispiel eines derartigen physikalischen Verfahrens ist die Raman-Spektroskopie. Im Folgenden werden hierzu kurz einige Grundzüge erläutert:
Um bei Molekülen Raman-Spektroskopie anwenden zu können, muss sich die Polarisierbarkeit bei Rotation oder Schwingung des Moleküls ändern. Bei der Raman-Spektroskopie wird die zu untersuchende Materie mit monochromatischem Licht bestrahlt, üblicherweise aus einem Laser. Im Spektrum des an der Probe gestreuten Lichts werden neben der eingestrahlten Frequenz (Rayleigh-Streuung) noch weitere Frequenzen beobachtet. Die Frequenzunterschiede zum eingestrahlten Licht entsprechen den für das Material charakteristischen Energien von Rotations-, Schwingungs-, Phonon- oder Spin-Flip-Prozessen. Aus dem erhaltenen Spektrum lassen sich, ähnlich wie bei Spektren der Infrarotspektroskopie, Rückschlüsse auf die untersuchte Substanz ziehen. Die in einem Raman-Spektrum auftretenden Linien werden auch als Stokes-Linien oder Antistokes-Linien bezeichnet.

Der Grund liegt in einer Wechselwirkung des Lichtes mit der Materie, dem sogenannten RAMAN-Effekt, bei dem Energie vom Licht auf die Materie übertragen wird ("Stokes-Seite" des Spektrums), bzw. Energie von der Materie auf das Licht ("Anti-Stokes-Seite" des Spektrums). Da die Wellenlänge des Lichts, d. h. seine Farbe, von der Energie des Lichtes abhängt, bewirkt dieser Energieübertrag eine Verschiebung der Wellenlänge des gestreuten Lichtes gegenüber dem eingestrahlten Licht, die sogenannte Raman-Verschiebung. Aus dem Spektrum, d.h. der Frequenz und der zugehörigen Intensität, und der Polarisation des gestreuten Lichtes kann man u. a. folgende Materialeigenschaften ableiten: Kristallinität, Kristallorientierung, Zusammensetzung, Verspannung, Temperatur, Dotierung und Relaxation.

Die RAMAN-Streuung von Molekülen besitzt normalerweise einen sehr kleinen Streuquerschnitt von beispielsweise ca. 10⁻³⁰ cm². Der Streuquerschnitt ist ein Maß für die Wahrscheinlichkeit, dass das zu messende Molekül mit der einfallenden Strahlung bzw. dem einfallenden Teilchen interagiert. Daher ist bei kleinen Streuquerschnitten eine relativ hohe Konzentration an Molekülen oder eine hohe Laserintensität, d.h. eine hohe Teilchenanzahl, notwendig, um ein detektierbares Signal zu erhalten. Es ist somit für einige Moleküle nicht möglich, RAMAN-Spektren zu erhalten.

Um Materialien wie Kunststoffe aus unterschiedlichen Quellen und/oder mit unterschiedlicher Zusammensetzung aufbereiten zu können, sodass definierte Qualitätsmerkmale wie z.B. mechanische und/oder optische Eigenschaften für den Wiedereinsatz erzielt werden, ist neben den nötigen maschinentechnischen Voraussetzungen eine Analyse der eingehenden aber auch der verarbeiteten Materialien nötig.

Für eine derartige Analyse kommen vorzugsweise Messvorrichtungen zum Einsatz, die insbesondere
- absolute Werte ausgeben können,
- einfach zu kalibrieren, zu warten und zu bedienen sind,
- online in der Maschine integriert werden können,
- robust und für einen 365 Tage /24 h Betrieb ausgelegt sind,
- in die Maschinensteuerung integriert werden sollten,
- flexibel unterschiedliche unbekannte Materialkomponenten feststellen können.

Weiters sollte die Möglichkeit bestehen, mehrere Messstellen an der Anlage mit einem System abzudecken und wirtschaftliche Aspekte wie die Kosten einer Messvorrichtung sind ebenfalls von Bedeutung.

Die Analytik mit RAMAN-Spektroskopie bietet hier als zusätzliche Vorteile die Möglichkeit, Absolutmessungen und Untersuchungen von organischen und anorganischen Bestandteilen durchzuführen sowie einen einfachen Kalibrierprozess. Weiters ermöglicht die Raman-Spektroskopie auch mit geringen Vorkenntnissen über die Zusammensetzung des zu verarbeitenden Materials auszukommen. Dies ist speziell im Postkonsumerbereich von großer Bedeutung, wo es kaum vorstellbar ist, alle im realen Betrieb möglicherweise auftretenden Kombinationen von Materialien in Modellen abzubilden, was andere Messkonzepte jedoch als notwendige Basis benötigen.

Eine weitere Möglichkeit besteht im Einsatz der Nahinfrarot (NIR)-Spektroskopie als physikalische Methode zur Untersuchung z.B. des Energiespektrums einer Probe anhand von Strahlung bzw. Wellen: Steigende Anforderungen an die Qualität von Kunststoffprodukten, sowie die Notwendigkeit einer Reduktion von Kosten bei der Herstellung und Aufbereitung, erfordern den Einsatz von schnellen und zuverlässigen Kontrollmethoden, die so früh wie möglich prozessrelevante Qualitätsparameter erfassen. Eine Methode, die dies leisten kann, ist die schnelle Nahinfrarot (NIR)-Spektroskopie.

Der Messaufbau für eine NIR-Spektroskopie lässt sich auch im Industrieumfeld mit geringem Aufwand implementieren. Es ist keine spezielle Probenaufbereitung notwendig und die Messung selbst erfolgt zerstörungsfrei. Es können Messungen am Granulat, an Pulver oder auch an fertigen Teilen durchgeführt werden.

Weiters kann mit dieser Methode der Polymeranteil bereits in der Preconditioning Unit PCU bzw. dem Schneidverdichter einer Qualitätskontrolle unterzogen werden. Diese Kontrolle liefert z.B. Aussagen über die Zusammensetzung von Polymerblends oder den Feuchtegehalt des Kunststoffes. Dadurch lassen sich Fehlchargen vermeiden und Qualitätsmerkmale kontinuierlich dokumentieren.

Die NIR-Technologie wird derzeit in der Kunststoff-Branche nur begrenzt beispielsweise in Sortierprozessen eingesetzt. Derzeit werden in den Unternehmen routinemäßig Eingangs- und Ausgangskontrollen durchgeführt. Diese Vorgehensweise verursacht hohe Kosten und ist mit einem hohen Zeitaufwand verbunden. Zusätzlich zu diesen Prüfungen sind auch oft Probenherstellung und -aufbereitung notwendig. Beim Einsatz der RAMAN- oder NIR-Technologie entfällt vorteilhafterweise die Probenvorbereitung. Weiters kann anhand eines, mit Hilfe von NIR-Spektroskopie erstellten, chemometrischen Modells einfach und in Sekundenschnelle eine Aussage über das zu prüfende Gut getroffen werden.

### Ausführungsbeispiele einer erfindungsgemäßen Vorrichtung

Aufgabe der Erfindung ist es, eine Inline-Prozesskontrolle an einer Produktionsanlage bzw. eine kontinuierliche Prozessüberwachung bei Extrusionsanlagen zu schaffen. Es sollen jene Materialeigenschaften ermittelt werden, die mit einer für den Anwender ausreichenden Genauigkeit reproduzierbar messbar sind.

Gewünschte Eigenschaften sind zB. folgende:
- Feuchtigkeit
- Additiv-/Füllstoffgehalt
- Farbgehalt qualitativ und quantitativ
- Polymerzusammensetzung, Comonomergehalt
- Detektion von Vernetzungen, resp. der Vernetzungsgrad (Gelierung)

Diese Parameter sollen gemessen und analysiert werden. Im Folgenden werden Ausführungsbeispiele einer erfindungsgemäßen Vorrichtung zum Aufbereiten, Bearbeiten und/oder Recyceln von Materialien, insbesondere von thermoplastischen Kunststoffmaterialien, beschrieben, bei denen mittels eines physikalischen Verfahrens wie RAMAN-Spektroskopie oder NIR-Spektroskopie inline Aussage über diese Parameter erhalten werden können.

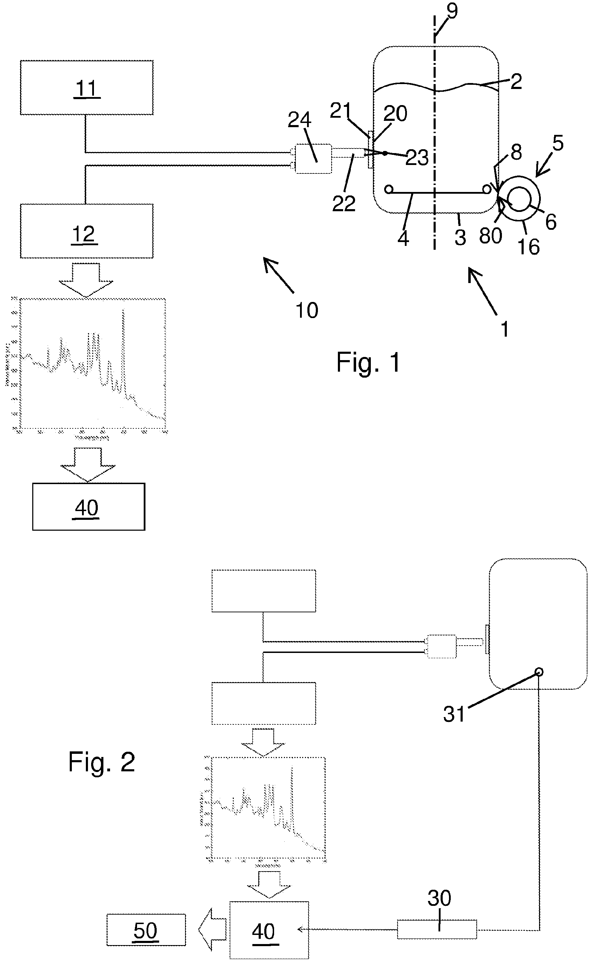

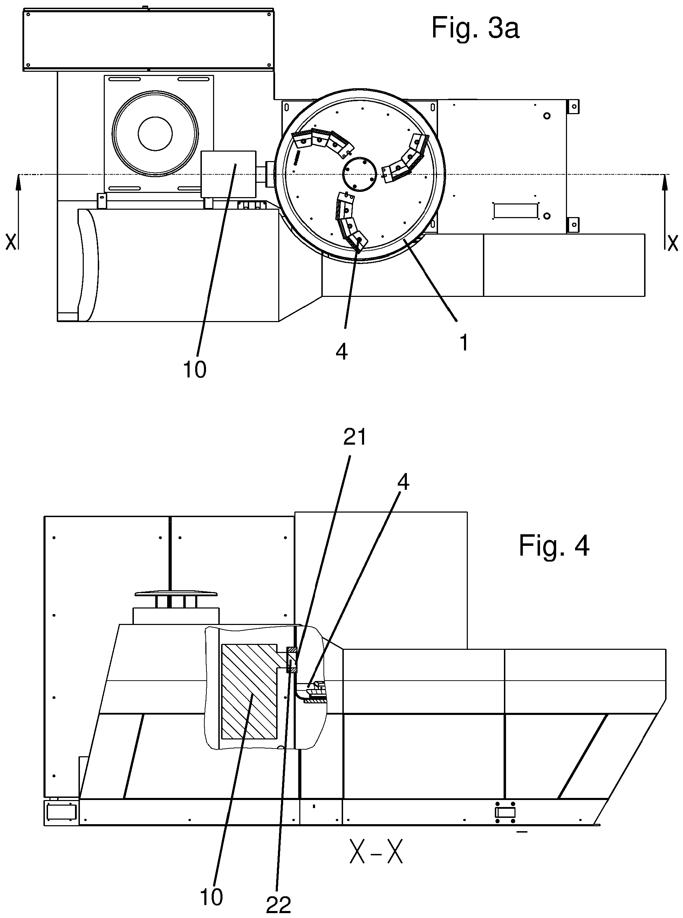

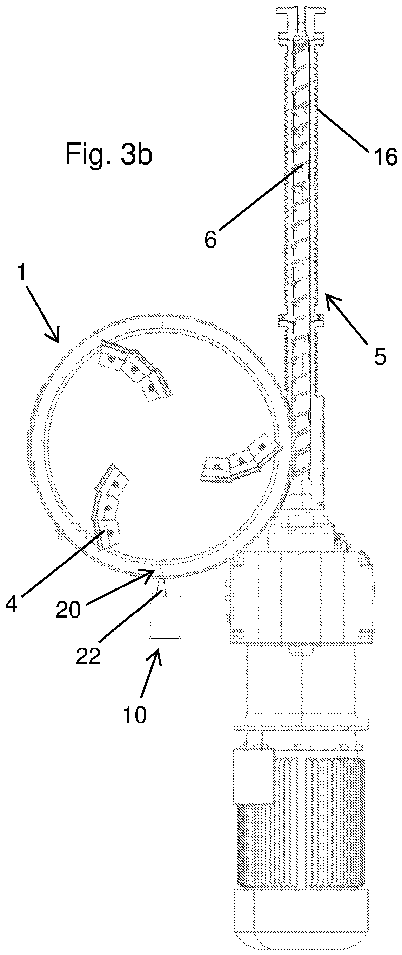

Zwei Ausführungsbeispiele einer erfindungsgemäßen Vorrichtung sind in größerem Detail in den Fig. 1 und Fig. 2, sowie in Fig. 3a, Fig. 3b und Fig. 4 dargestellt. Wie in Fig. 1 bis 4 ersichtlich ist, umfasst die Vorrichtung einen zuvor bereits beschriebenen Aufnahmebehälter 1, d.h. einen Schneidverdichter. Der Aufnahmebehälter 1 besitzt im gezeigten Ausführungsbeispiel eine Seitenwand 2, ist zylindrisch ausgebildet und weist eine untere Bodenfläche 3 auf. Alternativ dazu kann der Aufnahmebehälter 1 auch im Wesentlichen konisch ausgebildet sein, oder einen konischen oder zylindrischen Wandabschnitt aufweisen.

Der Aufnahmebehälter 1 umfasst weiters eine Misch- und/oder Zerkleinerungseinrichtung, die in der Nähe des Bodens 3 angeordnet ist. Im Ausführungsbeispiel in den Fig. 1 bis 4 weist der Aufnahmebehälter 1 als Misch- und/oder Zerkleinerungseinrichtung ein um eine vertikale Drehachse 9 drehbares, umlaufendes Misch- und/oder Zerkleinerungswerkzeug 4 auf. Das Misch- und/oder Zerkleinerungswerkzeug 4 dient zur Bewegung und Mischung, und gegebenenfalls auch zur Erwärmung, Zerkleinerung und/oder Erweichung, des zu behandelnden stückigen bzw. teilchenförmigen Materials, wobei im Betrieb im Aufnahmebehälter 1 eine Mischtrombe ausgebildet wird.

In einem erfindungsgemäßen Aufnahmebehälter 1 liegt das Material üblicherweise stückig bzw. teilchenförmig vor, z.B. als Mahlgut oder Folienschnitzel. Dabei haben derartige Folienschnitzel z.B. eine Dicke von ca. 10 µm, wobei schon hier einzelnen Polymerschichten im Folienaufbau vorhanden sein können, bis zu einigen mm. Die Folienschnitzel kann man sich dabei als tendenziell eher flächige Gebilde vorstellen. Die beiden anderen Dimensionen können von einigen mm bis ca 30 mm bis 500 mm gehen. Sie können aber auch nur einige mm betragen. Die Größe wird im Wesentlichen durch die Vorbehandlung bestimmt.

Die Mahlgüter können Dimensionen von mm bis ca. 30 mm bis 50 mm haben. Häufig bilden sich Würfel oder kugelähnliche oder sphärische Gebilde aus. Allerdings können auch Stäube oder kleinere Gebilde wie Mikrogranulat oder Granulat zum Einsatz kommen. Wesentlich ist, dass sich die Materialien ähnlich wie ein Fluid verhalten und von dem Misch- und/oder Zerkleinerungswerkzeug 4, das eine Umfangsgeschwindigkeit von ca. 1 m/s bis 100 m/s hat, in Form einer Trombe im Umlauf gehalten werden. Die Umfangsgeschwindigkeit der Misch- und/oder Zerkleinerungswerkzeug 4 ist vorzugsweise so gewählt, dass das stückige bzw. teilchenförmige Material radial mit einer Geschwindigkeit von 0,3 m/s bis 45 m/s und/oder in vertikaler Richtung mit einer

Geschwindigkeit von 0,1 m/s bis 60 m/s umläuft.

Dabei befindet sich das Material im Wesentlichen im Bereich von 0-80% der Höhe des Aufnahmebehälters 1. Das Material hat dabei außen am Aufnahmebehälter 1, d.h. z.B. an der Seitenwand 2, sowohl eine Bewegungsrichtung, die in Umfangsrichtung als auch eine Bewegungsrichtung die vornehmlich auch aufwärtsgerichtet ist. Wesentlich ist, dass das Material an der Seitenwand 2, resp. an einer sich dort befindlichen Messposition, häufig und regelmäßig ausgetauscht wird.

Beispielsweise bei einer Folien- oder Faserverarbeitung hat das Material im Aufnahmebehälter 1 eine mittlere Verweilzeit von ca. 10 bis 15 Minuten. Das Material läuft ca. mit 15 m/s radial um. Dementsprechend wird ein gewisses Volumensegment zwischen 40 - 200 mal an einer Messposition an der Seitenwand 2 vorbeikommen. Aus diesem Grund sind sowohl Langzeitmessungen möglich, d.h. es wird während der Messung selbst integriert, als auch eine Vielzahl von Messungen in sehr kurzer Zeit, d.h. es können dann in der Auswertung statistische Methoden verwendet werden um die Aussagefähigkeit der Messung zu erhöhen, worauf im Folgenden noch näher eingegangen wird.

In Fig. 1 ist im Detail ersichtlich, dass in einer Seitenwand 2 des Aufnahmebehälters 1 für die Austragung, beispielsweise im Bereich der Höhe des Misch- und/oder Zerkleinerungswerkzeugs 4, eine Öffnung 8 ausgebildet ist. Durch diese Öffnung 8 wird das vorbehandelte Kunststoffmaterial aus dem Inneren des Aufnahmebehälters 1 ausgebracht. Sind mehrere Misch- und/oder Zerkleinerungswerkzeuge 4 im Aufnahmebehälter 1 angeordnet, kann die Öffnung 8 im Bereich des untersten, bodennächsten Misch- und/oder Zerkleinerungswerkzeugs 4 angeordnet sein.

Ein Förderer, beispielsweise ein Extruder 5, mit einer in einem Gehäuse 16 rotierenden, z.B. plastifizierenden oder agglomerierenden, Schnecke 6, nimmt im Ausführungsbeispiel das aus der Öffnung 8 austretende vorbehandelte Material auf. Das Gehäuse 16 des Förderers kann dabei, wie in Fig. 1 und 2, eine an seiner Stirnseite oder in seiner Mantelwand liegende Einzugsöffnung 80 für das von der Schnecke 6 zu erfassende Material aufweisen. Diese Einzugsöffnung 80 steht mit der Öffnung 8 in Verbindung, durch die das Material aus dem Inneren des Aufnahmebehälters 1 austritt.

Eine erfindungsgemäßen Vorrichtung umfasst weiters eine spektroskopische und/oder spektrometrische Messvorrichtung 10 zur Analyse des sich im Inneren des Aufnahmebehälters 1 bewegenden stückigen bzw. teilchenförmigen Materials bzw. zur Gewinnung von Informationen über das jeweils vermessene Material, insbesondere von quantitativen und/oder qualitativen Kenngrößen des jeweiligen Materials. Es handelt sich dabei eine Messvorrichtung 10, die auf einem zuvor beschriebenen physikalischen Verfahren zur Untersuchung des z.B. Energiespektrums einer Probe anhand von Strahlung bzw. Wellen basiert.

Ein Ausführungsbeispiel einer derartigen mit einem Aufnahmebehälter 1 kombinierten spektroskopischen und/oder spektrometrischen Messvorrichtung 10 ist in den Fig. 1 bis 4 dargestellt. Die Messvorrichtung 10 vermisst inline, d.h. im laufenden Verarbeitungsbetrieb, zumindest Teile des sich im Inneren des Aufnahmebehälters 1 bewegenden stückigen bzw. teilchenförmigen Materials.

Die Messvorrichtung 10 einer erfindungsgemäßen Vorrichtung gibt eine physikalische Einwirkung, wie beispielsweise elektromagnetische Strahlung, Schall, elektrische Spannungen, oder Magnetfelder, zur Anregung zumindest eines Teils des rotierenden stückigen bzw. teilchenförmigen Materials ab. Dies wird im gezeigten Ausführungsbeispiel durch eine ins Innere des Aufnahmebehälters 1 wirkende bzw. gerichtete Anregungsquelle 11 bewirkt. Optional können auch mehrere derartige Anregungsquellen 11 vorgesehen sein.

Im gezeigten Ausführungsbeispiel gibt eine Anregungsquelle 11 elektromagnetische Strahlung zur Anregung des Materials ab. Die als Reaktion auf die Einwirkung entstehenden Messsignale, wie beispielsweise charakteristische Spektren der am vermessenen Material gestreuten elektromagnetischen Strahlung, werden von der Messvorrichtung 10 detektiert. Dazu umfasst die Messvorrichtung 10 zumindest einen Detektor 12 zur Erfassung der als Reaktion auf die Einwirkung entstehenden Messsignale, insbesondere charakteristischer Spektren der am vermessenen Material gestreuten elektromagnetischen Strahlung. Im Ausführungsbeispiel in den Fig. 1 bis 4 handelt es sich beim Detektor 12 um ein Spektroskop.

In den Fig. 1 und 2 ist die konstruktive Anbindung eines RAMAN-Spektrometers als Messvorrichtung 10 an einen Aufnahmebehälter 1 bzw. eine Preconditioning Unit PCU dargestellt. Dabei ist eine RAMAN-Sonde als ein Messkopf 24 mit einem Linsensystem 22 seitlich an den Aufnahmebehälter 1 angebunden, in einer Höhe unterhalb des üblichen Materiallevels bzw. Füllniveaus der im Aufnahmebehälter 1 bewegten Teilchen. Wie in Fig 1 und 2 ersichtlich ist, sind im Messkopf 24 sowohl der Lichtausgang für das von der Anregungsquelle 11 abgegebene Licht, als auch der Detektionseingang des Detektors 12 für das gestreute Licht aus dem Aufnahmebehälter 1 kombiniert.

Der Strahlweg des von der Anregungsquelle 11 abgegebenen Lichts in Richtung des Innenraums des Aufnahmebehälters 1 und der Strahlweg für das gestreute Licht aus dem Aufnahmebehälter 1 in Richtung des Detektors 12 können jedoch auch getrennt voneinander realisiert sein. Es ist auch möglich, dass der Detektor 12 im Messkopf 24 integriert ist. In diesem Fall kann eine Kühlung des Messkopfs 24 vorgenommen werden.

Alternativ dazu kann für die spektroskopische und/oder spektrometrische Analyse des Materials im Aufnahmebehälter 1 die Messvorrichtung 10 auch einen Detektor 12 für Atomspektroskopie oder Molekülspektroskopie, insbesondere eine Vorrichtung zur Raman-Spektroskopie, NIR-Spektroskopie, UV/VIS-Spektroskopie, Fluoreszenz-Spektroskopie und/oder Absorptions-Spektroskopie umfassen.

Im Ausführungsbeispiel in den Fig. 1 bis 4 umfasst die Messvorrichtung 10 weiters eine mit der Messvorrichtung 10, konkret der Anregungsquelle 11 und dem Detektor 12, in Datenkommunikation stehende Verarbeitungs- und Steuereinheit 40. Die Verarbeitungs- und Steuereinheit 40 steuert einerseits die Messvorrichtung 10 zur Abgabe der physikalischen Einwirkung, insbesondere zur Abgabe elektromagnetischer Strahlung, an. Andererseits steuert die Verarbeitungs- und Steuereinheit 40 die Messvorrichtung 10 dazu an, die entstehenden Messsignale, insbesondere die charakteristischen Spektren der gestreuten elektromagnetischen Strahlung, zu detektieren und die derart ermittelten Messwerte zur Verfügung zu halten.

Wie in Fig. 1 und Fig. 2 schematisch im Detail dargestellt ist, sind die Anregungsquelle 11, der Detektor 12, und die Verarbeitungs- und Steuereinheit 40 vibrationsfrei über faseroptische Systeme und/oder Lichtleiter 14 an den Aufnahmebehälter 1 angekoppelt. Die Anregungsquelle 11, der Detektor 12, und die Verarbeitungs- und Steuereinheit 40 können alternativ dazu auch physisch beabstandet vom Aufnahmebehälter 1 oder z.B. mittels Haltevorrichtungen am Aufnahmebehälter 1 angeordnet sein.

Kommt als Anregungsquelle 11 z.B. ein Laser zum Einsatz, so ist es vorteilhaft, wie im Ausführungsbeispiel in den Fig. 1 und 2, den Detektor 12 mittels Faseroptik vom Aufnahmebehälter 1 zu entkoppeln. Sollte keine Faseroptik für Laser bzw. Anregungsquelle 11 und Detektor 12 verwendet werden (Freistrahlsystem) so soll durch Beabstandung eine vibrationsfreie Ankopplung der Sensorik erreicht werden.

Beim Einsatz einer Faseroptik wird es erleichtert, die die Verarbeitungs- und Steuereinheit 40 und die Sensorik bzw. den Detektor 12 vom Aufnahmebehälter 1, der Temperaturschwankungen, Vibrationen usw. unterliegt, abzukoppeln. Die Faseroptik ist im Ausführungsbeispiel dabei so gewählt, dass sie eine Länge von kleiner 100 m, beispielsweise kleiner als 30 m bis 50 m vorzugweise kleiner als 15 m, hat, um eine entsprechende Dämpfung der Signale zu minimieren und somit vorteilhafterweise auch zu einer Kostenersparnis führt, da geringere Laserstärken erforderlich sind und das Signal/Rauschverhältnis verbessert wird.

Optische Elemente und die bildgebenden Systeme werden bevorzugt durch geeignete Maßnahmen wie zB. Luftspülung, Trockenluft, N₂-Spülung, etc. vor Umwelteinflüssen wie Staub, Feuchte, Temperatur, Sublimaten, etc. geschützt.

Im Aufnahmebehälter 1 ist zumindest eine Messöffnung 20 angeordnet, durch die eine Vermessung der Materialien im Inneren des Aufnahmebehälters 1 ermöglicht wird. Wie in den Fig. 1 und 2 im Detail ersichtlich ist, befindet sich diese im Ausführungsbeispiel in der Seitenwand 2 des Aufnahmebehälters 1. Durch die Messöffnung 20 wirkt die von der Anregungsquelle 11 abgegebene physikalische Einwirkung, im Ausführungsbeispiel ist dies die abgegebene elektromagnetische Strahlung, auf das Material im Inneren des Aufnahmebehälters 1 ein.

Die Messöffnung 20 kann einen Durchmesser von 0,5 mm bis 100 mm haben und durch ein Fenster 21 aus, für die physikalische Einwirkung wie z.B. die elektromagnetische Strahlung, durchlässigem Material, beispielsweise aus Saphirglas, verschlossen sein. Ein derartiges Fenster 21 bewirkt eine effektive Trennung zwischen der Sensorik, d.h. der Messvorrichtung 10 und dem zu vermessenden Material.

Die Messöffnung 20 ist im Ausführungsbeispiel in Fig. 1 bis 4 in der Seitenwand 2 des Aufnahmebehälters 1 im Bereich des unteren Drittels der Höhe des Aufnahmebehälters 1 angeordnet. Alternativ dazu kann die Messöffnung 20 auch im Bereich der Höhe des untersten, bodennächsten Misch- und/oder Zerkleinerungswerkzeugs 4, insbesondere etwas oberhalb oder unterhalb davon, vorzugsweise außerhalb des engsten Abstandes zwischen dem äußersten Punkt des Misch- und/oder Zerkleinerungswerkzeugs 4 und der Seitenwand 2 angeordnet sein.

Eine bevorzugte Aufbauposition der Messvorrichtung 10 ist seitlich am Aufnahmebehälter 1, da an dieser Position die kürzesten Abstände zum zu vermessenden Material realisierbar sind. Die Distanz zwischen anregendem System, d.h. der Anregungsquelle 11, wie z.B. einem Laser oder einer NIR-Quelle, und dem messenden System, d.h. dem Detektor 12 und zu vermessenden Material soll bevorzugt so gering wie möglich sein. Auf diese Weise können vorteilhafterweise einerseits die Anregungsquelle 11, d.h. die Lichtquellen, klein gehalten werden, während gleichzeitig ein gutes Messsignal gewährleistet ist, da Lichtintensität mit 1/r² abnimmt, wobei *r* den Abstand zwischen dem Detektor 12 und dem zum vermessenden Material angibt.

Beim seitlichen Aufbau am Aufnahmebehälter 1 wird bevorzugt jene Position gewählt, wo die Dichte des Materials, als auch der Druck auf die Seitenwand am höchsten ist. Ein bevorzugtes Ausführungsbeispiel eines erfindungsgemäßen Verfahrens zum Aufbereiten, Bearbeiten und/oder Recyceln von thermoplastischen Kunststoffmaterialien, mit einer am Aufnahmebehälter 1, d.h. dem Schneidverdichter, angeordneten Messvorrichtung 10, z.B. einem RAMAN-Spektrometer, wird im Folgenden beschrieben:
Das zu vermessende Material soll in ausreichender Häufigkeit an der Sensorik der Messvorrichtung 10 vorbeikommen, wobei das Material den Fokuspunkt 23 der Anregungsquelle 11 passieren soll. Zur Fokussierung der elektromagnetischen Strahlung der Anregungsquelle 11 auf einen Fokuspunkt 23 ist im Ausführungsbeispiel, siehe Details in den Fig. 1 und 2, eine Linse bzw. ein Linsensystem 22 vorgesehen. Der Fokuspunkt 23 liegt dabei am oder unmittelbar hinter dem Fenster 21, vorzugsweise in einem Abstand von maximal 10 cm hinter dem Fenster 21. Je höher dabei der Anpressdruck, den das rotierende Material auf den Aufnahmebehälter 1 bzw. eine Seitenwand 2 des Aufnahmebehälters 1 ausübt, - speziell bei Materialien mit geringer Schüttdichte - an diesem Fokuspunkt 23 ist, desto höher ist die Intensität des Messsignals, da das Material im Fokuspunkt 23 liegt.

Würde der Fokuspunkt 23 weiter hinten liegen, d.h. weiter im Inneren des Aufnahmebehälters 1 bzw. im Material, würden Streueffekte und verringerte Intensität das Signal negativ beeinflussen und das Signal/Rausch-Verhältnis entsprechend abnehmen. Aus diesem Grund ist der seitliche Einbau in den Aufnahmebehälter 1 vorteilhaft, und vorzugsweise, wie bereits zuvor beschrieben, im unteren Bereich des Aufnahmebehälters 1.

Vorteilhaft ist es, wenn das Verfahren im Aufnahmebehälter 1 so geführt wird, dass das Niveau der Materialteilchen bzw. der durch die Bewegung ausgebildeten Mischtrombe im Aufnahmebehälter 1 so gehalten wird, dass es ständig oberhalb der Anregungsquelle 11 bzw. der Lichtquelle liegt.

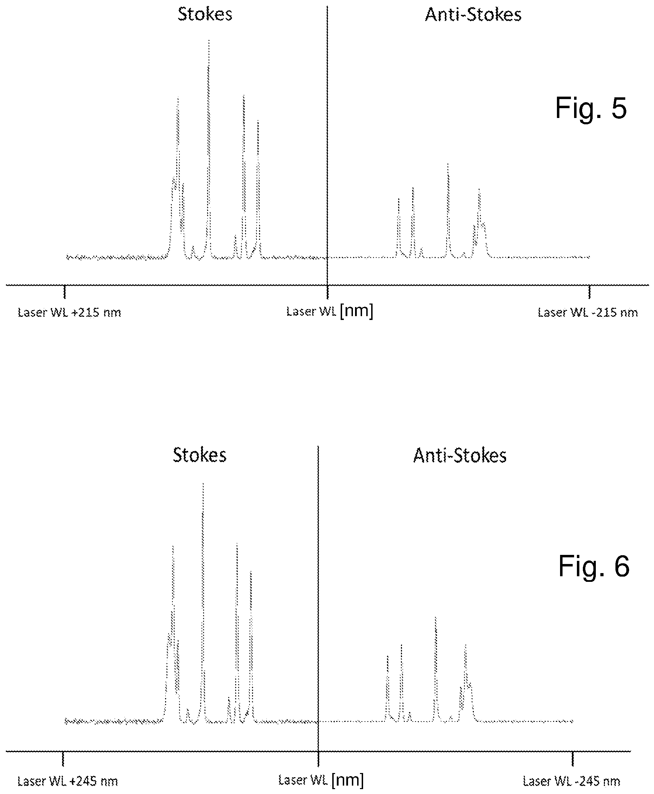

Bei einer erfindungsgemäßen Vorrichtung bedeckt das Material bei den Betriebsbedingungen das Fenster 21 somit ständig. Diese Bedeckung dient auch zur Abschirmung von Fremdlicht im Frequenzbereich von 570 nm bis 1008 nm, insbesondere von 785+/-215 nm, in dem die Messfrequenzen liegen. Dabei handelt es sich um 785 + 215 nm für den Stokes-Bereich und 785 - 215 nm für den Antistokes-Bereich, siehe Fig. 5. Beim Raman-Effekt kommt es sowohl zu einem Energieübertrag vom Photon (Stokes-Streuung) auf das Molekül bzw. vom Molekül auf das Photon (Anti-Stokes-Streuung). Beide Übergänge können einzeln oder/und in einem Verhältnis zueinander ausgewertet werden. Weiters ist bei einem weitgehend ständig bedeckten Messkopf 24/Fenster 21 vorteilhafterweise eine gute Stabilität des Messsignals während der Messzeit zu erzielen. Optional dazu ist auch ein Bereich von +/- 245 nm möglich, siehe Fig. 6.

Wie bereits zuvor erwähnt, ist das Fenster im Ausführungsbeispiel in Fig. 1 bis 4 vorteilhafterweise aus Saphirglas gebildet, um die relevanten Frequenzspektren im Bereich von 570 nm bis 1008 nm, insbesondere von 785+/-215 nm passieren zu lassen. Um Anwendung im Wellenlängenbereich von 785 nm abzudecken, sollte mind. der Bereich zwischen 931 nm (Stokes) und 678 nm (Anti-Stokes) durchgelassen werden.

Die Flächen des Fensters 21 sind im Ausführungsbeispiel planeben und zueinander parallel ausgerichtet. Optional kann die dem Aufnahmebehälter 1 zugewendete inneren Fläche des Fensters 21 auch konkav dem Radius des Aufnahmebehälters 1 angepasst und die dem Aufnahmebehälter 1 abgewendete äußere Fläche des Fensters 21 parallel konkav zur inneren Fläche ausgebildet sein.

Wie bereits zuvor erwähnt, kann die Anregungsquelle 11 wie im gezeigten Ausführungsbeispiel ein Laser sein mit einem Wellenlängenbereich von 100 nm bis 1400 nm, im Bereich des infraroten, des sichtbaren und/oder des UV-Lichts, z.B. einem Wellenlängenbereich von 780+/-250 nm. Weitere mögliche einsetzbare Laser können einen Wellenlängenbereich von 532 +/- 215nm, 638 +/- 215nm, 820 +/- 215nm und/oder 1064 +/-215nm abdecken.

Die Laserleistung liegt im Bereich von 15 mW bis 5 W, wobei 100 mW bis 500 mW bevorzugt werden. Der Einsatz von Lasern mit hoher Energiedichte ist vorteilhafterweise möglich, da das Material ständig ausgetauscht wird und es dadurch zu keiner Veränderung des zu messenden Materials kommt. Besondere Notwendigkeit von hohen Energiedichten kann es z.B. bei sehr dunkel eingefärbten Polymeren geben. Weiter ist auf ein Verhältnis Laserleistung zu Integrationszeit zu achten. Die liegt bevorzugt im Bereich von 5 mW/s bis 5000 mW/s, insbesondere im Bereich von 15 mW/s bis 1000 mW/s.

Es empfiehlt sich den an der Messöffnung 20 bzw. dem Fenster 21 angeordneten Messkopf 24 der Messvorrichtung 10 mit dem Linsensystem 22 zu kühlen, um dauerhaft unter 90°C, besser unter 60°C, zu bleiben. Dabei können Gase oder Flüssigkeiten als Kühlmedien eingesetzt werden, es kann aber auch ein Peltier-Element zum Einsatz kommen.

Bei Verwendung von Nahinfrarot gelten vorzugsweise ebenso die zuvor beschriebenen Bedingungen. In diesem Fall kann das Fenster allerdings auch aus Quarzglas bestehen, um die relevanten Frequenzspektren im Bereich von 760 nm bis 2500 nm für NIR passieren zu lassen.

Bei gleichzeitigem Aufbau von Messvorrichtungen 10 basierend auf Raman-Spektroskopie bzw. Nahinfrarot-Spektroskopie sind Faseroptiksysteme zu bevorzugen, um den Aufbau einfach zu halten. Faseroptiksystem benötigen unmittelbar vor dem Fenster 21 den geringsten Platz. Dabei ist es möglich beide Messvorrichtungen 10 über ein Fenster 21 einzukoppeln, aber auch über unterschiedliche Fenster 21. Dabei hat es sich als sinnvoll erwiesen, wenn sich die Fenster 21 weitgehend auf der gleichen Höhe in Umfangsrichtung befinden. Eine örtliche Nähe ist anzustreben, aber nicht notwendig.

Der Volumenbereich des Materials, der untersucht werden kann, wird definiert durch eine Messfleck-Querschnittsfläche im Bereich von 0,1 mm bis 5 mm, insbesondere von 1 mm bis 3 mm, und eine Eindringtiefe in das Material von 0,3 µm bis zu 30 µm. Die Eindringtiefe, die in der Praxis zu guten Messwerten führt, liegt im Bereich von 8 µm bis 15 µm. Die Messfleck-Querschnittsfläche beträgt im Ausführungsbeispiel ca. 1 mm bis 3 mm, sodass ein Volumen von ca. 0,00015 mm³ untersucht werden kann. Aus diesem Grund ist die Häufigkeit der Messung bzw. das häufige Vorbeiwandern des zu vermessenden Materials am Fenster 21 wichtig, um repräsentative Messergebnisse zu erzielen.

Dadurch dass das Material vor dem Fenster 21 bzw. im Fokuspunkt 23 sich im Betrieb häufig und regelmäßig austauscht und durch eine angepasste Häufigkeit der Messungen bzw. durch die entsprechende Messdauer ist eine extrem hohe Genauigkeit erzielbar.

Die Verarbeitungs- und Steuereinheit 40 leitet auf Grundlage der ermittelten Messwerte Informationen über das jeweils vermessene Material, insbesondere quantitative und/oder qualitative Kenngrößen des jeweiligen Materials, ab und hält diese zur Verfügung. Beispielsweise kann die Verarbeitungs- und Steuereinheit 40 die als Reaktion auf die Einwirkung entstehenden Messsignale, insbesondere von charakteristischen Spektren der am vermessenen Material gestreuten elektromagnetischen Strahlung, spektrometrisch und/oder spektroskopisch analysieren.

### Verfahrensführung und Auswertung

Wir bereits zuvor beschrieben, wird das sich im Inneren des Aufnahmebehälters 1 bewegende stückige bzw. teilchenförmige Material inline spektroskopisch und/oder spektrometrisch analysiert oder gemessen und die derart ermittelten Messwerte werden zur Gewinnung von Informationen über das jeweils vermessene Material, insbesondere von quantitativen und/oder qualitativen Kenngrößen des jeweiligen Materials, herangezogen. Im Ausführungsbeispiel werden die als Reaktion auf die elektromagnetische Strahlung entstehenden Messsignale, in Form von charakteristischen Spektren der am vermessenen Material gestreuten elektromagnetischen Strahlung, vorzugsweise spektrometrisch, detektiert und ausgewertet werden.

Die Auswertung der so erhaltenen Messergebnisse bzw. Spektren wird von der Verarbeitungs- und Steuereinheit 40 durchgeführt und erfolgt vorteilhafterweise wie folgt:
Durch das häufige Vorbeiströmen der Materialteilchen in festen bis teilerweichten Zustand an der Messposition bzw. dem Fokuspunkt 23 ist es möglich, lange Messzeiten zu verwenden, die den Einsatz von z.B. Lasern mit geringer Leistung von ca. 20 - 200 mW ermöglichen, während die Aussagefähigkeit der Messergebnisse ausreichend genau bleibt.

Die Verarbeitungs- und Steuereinheit 40 steuert in diesem Fall die Messvorrichtung 10, bzw. die Anregungsquelle 11, derart, dass diese die physikalische Einwirkung, beispielsweise die vom Laser abgegebene elektromagnetische Strahlung, während eines vorgegebenen Zeitraums, z.B. von weniger als einer Sekunde bis zu mehreren Sekunden oder einer Minute oder mehr, kontinuierlich abgibt. Anschließend berechnet die Verarbeitungs- und Steuereinheit 40 für den jeweiligen Zeitraum eine einzelne gemeinsame Information über das jeweils vermessene Material, d.h. alle Materialteilchen, die in diesem Zeitraum die Messöffnung 20 bzw. das Fenster 21 passiert haben und vom Detektor 12 erfasst wurden. So kann beispielsweise ein einzelnes Summenspektrum für alle diese Materialteilchen erstellt werden.

Alternativ dazu kann die Verarbeitungs- und Steuereinheit 40 die Messvorrichtung 10, bzw. die Anregungsquelle 11, derart steuern, dass sie zu einer Vielzahl an vorgegebenen Zeitpunkten die physikalische Einwirkung, beispielsweise die vom Laser abgegebene elektromagnetische Strahlung, wiederholt abgibt. Somit wird bei jeder Abgabe jeweils ein Teilchen des im Aufnahmebehälter 1 rotierenden Materials angeregt und streut Strahlung zurück, die vom Detektor 12 detektiert wird. Anschließend kann die Verarbeitungs- und Steuereinheit 40 einen Mittelwert der Informationen über das jeweils vermessene Material, auf Grundlage ausgewählter, oder aller, zu diesen Zeitpunkten von der Messvorrichtung 10, insbesondere dem Detektor 12, für einzelne Teilchen ermittelten Messwerte berechnen und zur Verfügung zu halten. Dies bedeutet, dass ein Mittelwert aus einer Vielzahl an Spektren einzelner Teilchen gebildet wird.

Sollten wegen der zu vermessenden Materialien hohe/höhere Laserleistungen nötig sein, verhindert das Vorbeiströmen des Materials eine Beeinflussung des zu messenden Materials durch die hohe Energiedichte des Lasers am Fokuspunkt 23. Methodisch wird ein unbekanntes Material mit höher Energiedichte angeregt und die Verarbeitungs- und Steuereinheit 40 regelt optional die Laserleistung so lange nach unten, bis sich der Detektor 12 in seinem linearen Bereich befindet. In einem statischen Messvorgang könnte dieser automatisierte Messvorgang nicht verwendet werden, da die Probe bzw. das Material verbrennen oder schmelzen würde.

Da im Aufnahmebehälter 1, d.h. dem Schneidverdichter, üblicherweise eine Temperaturveränderung des zu verarbeitenden Materials erfolgt, kann eine erfindungsgemäße Vorrichtung optional auch eine Temperaturmesseinrichtung 30 umfassen, die der Verarbeitungs- und Steuereinheit 40 vorgeschaltet ist.

Bei einer üblichen Betriebsweise des Aufnahmebehälters 1 erfolgt jedenfalls eine Temperaturänderung des Materials: das Material wird bei Raumtemperatur zugeführt bzw. oben in den Aufnahmebehälter 1 eingebracht und wird dann zB durch die Bewegung der Misch- und/oder Zerkleinerungswerkzeuge 4 bzw. durch Reibung erwärmt. Das Material wird heiß, erweicht, bleibt allerdings immer teilchenförmig bzw. stückig und schmilzt nicht. Diese Temperaturänderung führt allerdings zu einer Veränderung der von der Messvorrichtung 10 bzw. dem Detektor 12 aufgenommenen Spektren durch die strukturelle Änderung der Polymere in diesem Prozessschritt. Daher ist es vorteilhaft, wenn bei der Auswertung eine entsprechende Korrektur vorgenommen wird, speziell wenn das jeweils erfasste Spektrum gegen bestehende Spektrendatenbanken abgeglichen werden soll. Eine derartige optionale Temperaturmesseinrichtung 30 misst die Temperatur im Inneren des Aufnahmebehälters 1 und/oder die Temperatur des Materials und übermittelt diese an die Verarbeitungs- und Steuereinheit 40. Dabei wird z.B. mit einem Temperaturfühler, der in die Randschicht des Materials ragt, die Materialtemperatur erfasst und als Vorgabe für die Korrektur der Spektren verwendet. Alternativ kann auch die Behältertemperatur des Aufnahmebehälters 1 als Wert herangezogen werden. Es kann auch ein weiteres thermisches Messgerät z.B. optischer Natur entsprechend am Aufnahmebehälter 1 angebracht werden, um die Temperatur zu erfassen. Besonders vorteilhaft ist es in diesem Zusammenhang, den Einbauort der Temperaturmesseinrichtung 30 in örtlicher Nähe zum, die Messöffnung 20 bedeckenden, Fenster 21 bzw. zum Messkopf 24 der Messvorrichtung anzulegen. Die Temperaturmesseinrichtung 30 kann im Aufnahmebehälter 1 z.B. auf gleicher Höhe, insbesondere an gleicher Position, wie die zumindest eine Messöffnung 20 angeordnet sein.

Die Verarbeitungs- und Steuereinheit 40 zieht die von der einen Temperaturmesseinrichtung 30 ermittelten Messwerte für eine Korrektur des Temperatureinflusses auf die für das jeweils vermessene Material ermittelten Informationen, insbesondere der temperaturabhängigen charakteristischen Spektren der am vermessenen Material gestreuten elektromagnetischen Strahlung, heran und hält die derart korrigierten Informationen, insbesondere Spektren, zur Verfügung.

So werden die derart gewonnenen Temperaturinformationen in die Auswertung einbezogen und dienen als Indikation für eine Temperatur-Korrektur der Spektren. So wird beispielsweise die Materialtemperatur des Materials im Aufnahmebehälter 1 gemessen und an die Verarbeitungs- und Steuereinheit 40 geschickt. Dort wird die gemessene Ist-Temperatur zur Korrektur der Spektren z.B. auf eine Referenztemperatur herangezogen, um einen einfachen Vergleich mit hinterlegten Referenzspektren zu ermöglichen.

Beispielsweise wird ein durch die Temperatur bedingter Shift, d.h. eine Verschiebung, der Spektren zu höheren Intensitäten korrigiert.

Für eine besonders einfache Auswertung der für das vermessene Material erhaltenen Informationen wie Spektren kann die Verarbeitungs- und Steuereinheit 40 einen Speicher umfassen, in dem Referenzinformationen, z.B. quantitative und/oder qualitative Referenzkenngrößen oder Referenzspektren, hinterlegt sind. Die Verarbeitungs- und Steuereinheit 40 kann dann die für das jeweils vermessene Material ermittelten Informationen wie Spektren, mit den Referenzinformationen z.B. Referenzspektren, vergleichen und die Abweichung von den Referenzinformationen bzw. Referenzspektren, bestimmen. Diese ermittelte Abweichung kann anschließend z.B. an eine die Prozess-Steuerungseinheit und/oder eine Anzeigeeinheit weitergeleitet werden.

Die Verarbeitungs- und Steuereinheit 40 kann optional auch mit einer Prozess-Steuerungseinheit 50 zusammenwirkt bzw. in Datenkommunikation stehen. Eine derartige Prozess-Steuerungseinheit kann beispielsweise von der Verarbeitungs- und Steuereinheit 40 übermittelte Daten, d.h. Informationen über das jeweils vermessene Material, wie quantitative und/oder qualitative Kenngrößen des jeweiligen Materials, zur Überwachung und/oder Steuerung der Prozessführung im Aufnahmebehälter 1 und/oder der nachfolgenden Prozesskette nutzen.

Eine derartige Prozess-Steuerungseinheit 50 kann beispielsweise auf Grundlage der von der Verarbeitungs- und Steuereinheit 40 übermittelten Daten
- eine Zudosierung von Füllstoffen in den Aufnahmebehälter 1 vornehmen und/oder
- eine Zuführung von Materialien, wie Polymeren, Füllstoffen, etc., in den Aufnahmebehälter 1 und/oder eine an den Aufnahmebehälter 1 angeschlossene Austragungsvorrichtung vornehmen und/oder
- verarbeitete Materialien, insbesondere Granulat, aus dem Aufnahmebehälter 1 mittels einer an den Aufnahmebehälter 1 angeschlossenen Austragungsvorrichtung wie dem Förderer ausschleusen. Im Folgenden wird darauf noch näher eingegangen.

### Anwendunasbeispiele

### Beispiel 1:

Im Folgenden wird ein erstes Anwendungsbeispiel eines erfindungsgemäßen Verfahrens bzw. einer erfindungsgemäßen Vorrichtung zum Aufbereiten, Bearbeiten und/oder Recyceln von thermoplastischen Kunststoffmaterialien, beschrieben:
Bei der Herstellung von Hygieneartikeln wie Windeln, Binden etc. kommen unterschiedliche Polymere mit unterschiedlichen Viskositäten und Füllgraden an Füllstoffen wir z.B. CaCO₃ zum Einsatz. Dabei besteht der Vliesanteil der Hygieneartikel meist aus ungefülltem PP und die Folie aus mit CaCO₃ gefülltem LD-PE. Die bei der Produktion anfallenden Stanzreste aber auch Folienreste haben daher einen unterschiedlichen Polymeranteil und einen unterschiedlichen Füllgrad.

Um nun aus diesen Ausgangsmaterialien einen Werkstoff mit gleichbleibender Qualität zu erzeugen, muss zwingend bekannt sein, wie hoch die unterschiedlichen Bestandteile an Füllstoff oder/und Polymer sind. In einem Compoundierschritt kann nun durch Beigabe von Polymer, Additiven und Füllstoff das Endprodukt auf eine definierte Mischung eingestellt werden, sofern die eingehenden Bestandteile absolut bekannt sind. Es wird dadurch die Flexibilität entsprechend erhöht. Eine Messung vor und zur Kontrolle nach dem Compoundierschritt steuert die Anteile der zugeführten Bestandteile und sichert somit gewisse Eigenschaften und minimiert die Kosten der Produktion.

### Versuchsaufbau

Bei dieser Versuchsdurchführung wurde eine INTAREMA^{®} 1180TVEplus umfassend eine Messvorrichtung 10 mit einem Detektor 12 für RAMAN-Spektroskopie und einen Aufnahmebehälter 1, d.h. einen Schneidverdichter bzw. eine Preconditioning Unit, mit Produktionsabfällen aus der Produktion von Hygienefolie beschickt. Dieses Material liegt in Form von stückigen Folienabschnitten vor. In Tabelle 1 sind die Versuchsdaten zusammengefasst.

Der in der folgenden Tabelle 1 verwendete Begriff "INTAREMA" bezieht sich auf "INTAREMA^{®}".

**Tabelle 1: Versuchsdaten erstes Anwendungsbeispiel**

| Material | Maschine | PCU Temperatur | PCU Leistung | Drehzahl Werkzeug | Laserintensität | Integrationszeit | Anzahl Messungen | Messzeit pro Spektrum |
|---|---|---|---|---|---|---|---|---|
| | | [°C] | [kW] | [rpm] | [mW] | [s] | | [s] |
| LDPE+ CaCO₃ | INTAREMA 1108 TVEplus | 102 | 36 - 38 | 750 | 200 | 3 | 10 | 30 |

### Auswertung

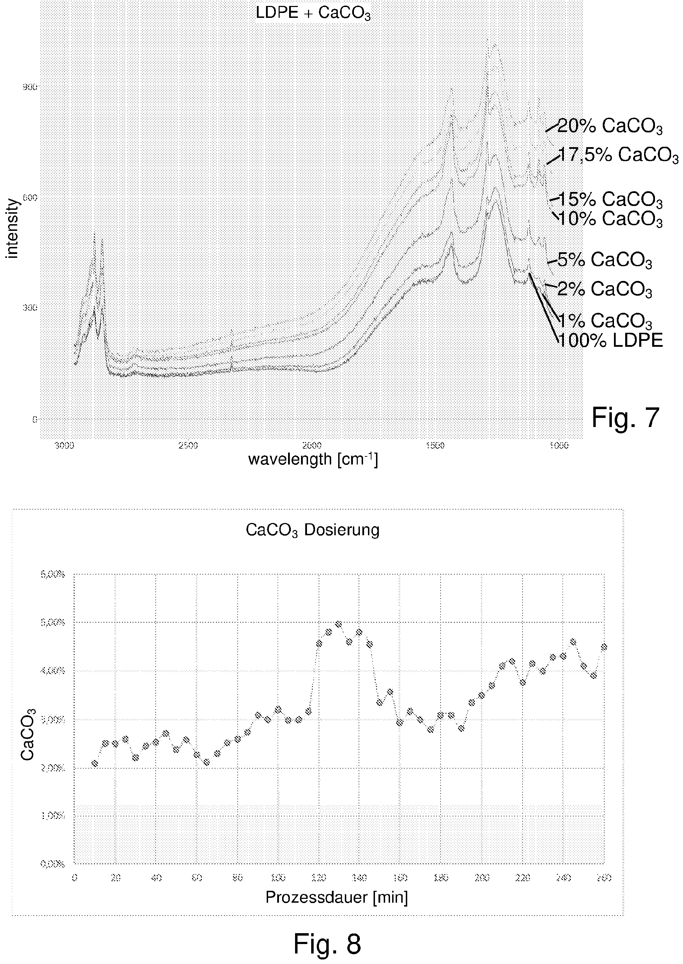

Überraschenderweise konnte trotz der zuvor bereits erwähnten hohen Umlaufgeschwindigkeiten der Teilchen im Aufnahmebehälter 1 vom Detektor 12 bzw. dem Spektroskop eindeutige Spektren registriert werden. Die vom Detektor 12 erfassten Spektren sind in Fig. 7 dargestellt. Die Spektren zeigen Mischungen aus PE und variierenden Prozentanteilen an CaCO₃ (1%, 2%, 5%, 10%, 15%, 17,5%, 20% CaCO₃) an.

Die erfassten Spektren werden von der Verarbeitungs- und Steuereinheit 40 hinsichtlich des Anteils an CaCO₃ ausgewertet und der Anteil des CaCO₃ wird in % des Gesamtmaterials von der Verarbeitungs- und Steuereinheit 40 zur Verfügung gestellt.

### Interpretation

In den Messergebnissen kann die Schwankung des CaCO₃-Anteils im Eingangsmaterial während der Prozessdauer von ca. 4h, siehe Fig. 8, erkannt werden. Somit kann ein Steuersignal an eine an die Verarbeitungs- und Steuereinheit 40 angeschlossene Prozess-Steuereinheit 50, wie z.B. eine Dosiereinheit, abgeleitet werden, auf Grundlage dessen die Zufuhr von CaCO₃ in Pulverform oder als Masterbatch entsprechend adaptiert wird. Bei höherem Anteil im Eingangsmaterial kann also die Dosiermenge entsprechend reduziert und bei geringerem Anteil im Eingangsmaterial erhöht werden. Im erzeugten Endprodukt liegt dann vorteilhafterweise ein gleichmäßiger CaCO₃-Anteil vor. Üblicherweise wird für Spritzgussprodukte ein CaCO₃ Anteil von ca. 15% - 25% je nach Anwendung vorgesehen, wobei die Abweichung jeweils nur +/- 1% betragen darf.

### Beispiel 2:

Im Folgenden wird ein zweites Anwendungsbeispiel eins erfindungsgemäßen Verfahrens bzw. einer erfindungsgemäßen Vorrichtung beschrieben:
Bei dem Wiedereinsatz von gebrauchten Verpackungsstoffen aus dem Food/NonFood-Bereich werden durch Sortier- und Waschprozesse die unterschiedlichen Polymerströme so gut wie möglich aufgetrennt und abgereinigt. Im darauffolgenden thermischen Umformungsprozess (Extrusion) wird sowohl eine Homogenisierung als auch eine Schmelzefiltration vorgenommen. Die daraus produzierten Regenerate müssen je nach Anwendung einer gewissen Qualität entsprechen: So soll zB zur Herstellung von Blasfolie aus LD-PE der Anteil von PP ein gewissen Prozentsatz nicht übersteigen, da dann in weiterer Folge die gewünschten mechanischen Eigenschaften unter Umständen nicht erreicht werden oder auch die Verschweißbarkeit nicht mehr gegeben sein kann. Da weiters in dem vorgelagerten Sortierprozess nie eine 100%ige Sortenreinheit erreicht werden kann, ist zB eine Ausschleusung von Granulaten aus einem, an den Aufnahmebehälter 1 angeschlossenen Extruder 5 mit höherem PP-Anteil sinnvoll. Durch eine Inline-RAMAN-Messung kann der PP-Anteil im Aufnahmebehälter 1 leichter festgestellt und eine Ausschleusung von fertig produzierten Granulaten angesteuert werden, wenn der PP-Anteil zu hoch ist.

### Versuchsaufbau

Bei der Versuchsdurchführung wurde eine INTAREMA^{®} 80TVEplus umfassend eine Messvorrichtung 10 mit einem Detektor 12 für RAMAN-Spektroskopie und einen Aufnahmebehälter 1, d.h. einen Schneidverdichter bzw. eine Preconditioning Unit, verwendet. Bei diesem Versuch wurde durch Zugabe von PE-Folie zu einer reinen PP-Folie in den Aufnahmebehälter 1 die Tauglichkeit einer Messvorrichtung basierend auf RAMAN-Spektroskopie nachgewiesen, die in vorgegebenen Zeitabständen wiederholt Messungen durchführt. Dazu wurde jeweils zwei Folienrollen, eine PP-Rolle und eine LDPe-Rolle, mit bekannter Folienbreite und Foliendicke über jeweils ein separates Einzugswert dem Aufnahmebehälter 1 zugeführt. Die prozentuelle Aufteilung wurde rechnerisch aus dem Flächengewicht ermittelt und durch Stichprobenprüfung am erzeugten Granulat erhärtet. In Tabelle 2 sind die Versuchsdaten zusammengefasst.

**Tabelle 2: Versuchsdaten zweites Anwendungsbeispiel (der in der Tabelle verwendete Begriff "INTAREMA" bezieht sich auf "INTAREMA^{®}")**

| Material | Maschine | PCU Temperatur | PCU Leistung | Drehzahl Werkzeug | Laser - intensität | Integrationszeit | Anzahl Messungen | Messzeit pro Spektrum |
|---|---|---|---|---|---|---|---|---|
| | | [°C] | [kW] | [rpm] | [mW] | [s] | | [s] |
| PE/PP | INTAREMA 1108 TVEplus | 100 | 40-41 | 750 | 150 | 4 | 10 | 40 |

### Auswertung

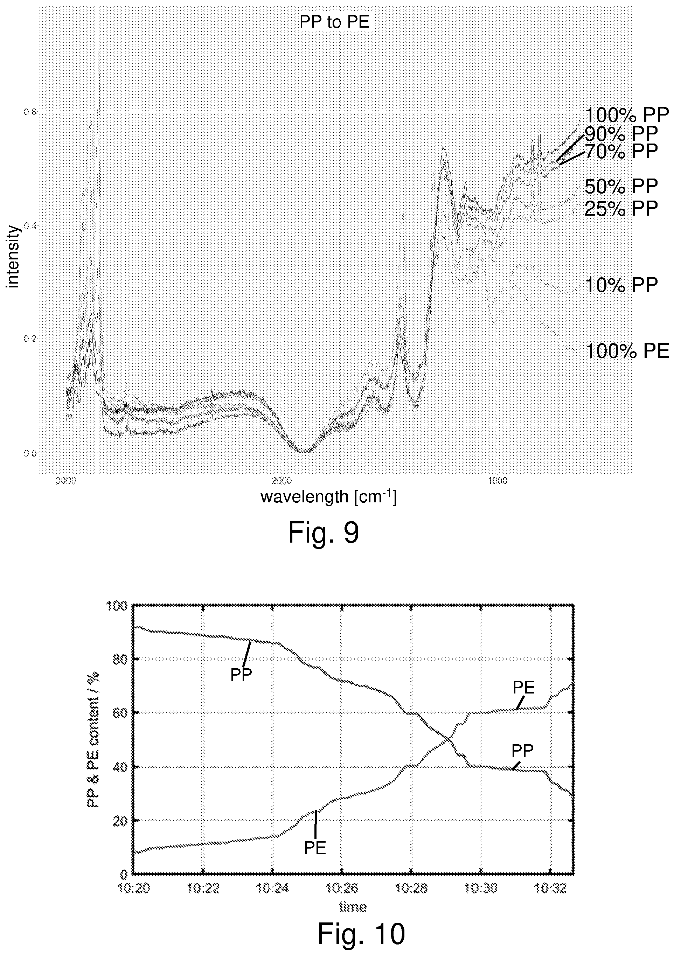

Die vom Detektor 12 erfassten Spektren sind in Fig. 9 dargestellt. Die Spektren zeigen Mischungen aus PP und PE mit jeweils variierenden Prozentanteilen (100%, 90%, 70%, 50%, 25%, 10% 0% PP).

Die erfassten Spektren werden von der Verarbeitungs- und Steuereinheit 40 ausgewertet und der Anteil des PP wird in % des Gesamtmaterials von der Verarbeitungs- und Steuereinheit 40 zur Verfügung gestellt, siehe Fig. 10.

### Interpretation

Durch den Einsatz dieses Messvorgangs ist es möglich, den PP-Anteil mit einer Genauigkeit von kleiner 1% im Materialstrom zu messen. Auf diese Weise ist es einfach möglich, den PP-Anteil im Prozessendprodukt durch Zugabe von PE-Material zu steuern. Dies ist besonders vorteilhaft, da aus dem praktischen Alltag bekannt ist, dass schon ein Anteil von ca. 5% PP im LD-Strom die Verschweißbarkeit so reduziert, dass eine Sackproduktion nicht mehr gegeben ist.

### Beispiel 3:

Im Folgenden wird ein drittes Anwendungsbeispiel eins erfindungsgemäßen Verfahrens bzw. einer erfindungsgemäßen Vorrichtung beschrieben:
PE/PA-Verbunde werden als Verpackungsfolie für Lebensmittel verwendet. Dabei handelt es sich um einen mehrschichtigen Aufbau, in dessen Mitte sich die PA-Folie, die als zB. Aromaschutz funktioniert, befindet. PE (Polyethylen) und PA (Polyamid) sind Polymere, die sich nicht gut mischen. Man kann aber durch Zugabe von Verträglichmachern einen sehr guten Werkstoff für die Folienproduktion oder den Spritzguss gewinnen. Da die Verträglichmacher sehr teuer sind und die Anteile des PA und PE schwanken, können bei der Aufbereitung Kosten gespart und das Prozessendprodukt optimiert werden, wenn der Polymeranteil genau bestimmt und die Zugabe des Verträglichmachers entsprechend gesteuert werden kann. Die Reststoffe haben meist einen Anteil von ca. 40% PA und 60% PE und liegen in flächigen Gebilden, Randstreifen und Folienrollen vor.

### Versuchsaufbau

Bei der Versuchsdurchführung wurde eine INTAREMA^{®} 1108 TE umfassend eine Messvorrichtung 10 mit einem Detektor 12 für RAMAN-Spektroskopie und einen Aufnahmebehälter 1, d.h. einen Schneidverdichter bzw. eine Preconditioning Unit, verwendet. Bei diesem Versuch wurde durch Zugabe der PE/PA-Randstreifen und Folienstücke in den Aufnahmebehälter 1 die Tauglichkeit einer kontinuierlich messenden Messvorrichtung 10 basierend auf RAMAN-Spektroskopie nachgewiesen. Im erzeugten Granulat wurden die entsprechenden Polymeranteile mit Labormessgeräten, zur Kontrolle der zuvor inline bestimmten Anteile, gemessen. In Tabelle 3 sind die Versuchsdaten zusammengefasst.

**Tabelle 3: Versuchsdaten drittes Anwendungsbeispiel (der in der Tabelle verwendete Begriff "INTAREMA" bezieht sich auf "INTAREMA^{®}")**

| Material | Maschine | PCU Temperatur | PCU Leistung | Drehzahl Werkzeug | Laserintensität | Integrationszeit | Anzahl Messungen | Messzeit pro Spektrum |
|---|---|---|---|---|---|---|---|---|
| | | [°C] | [kW] | [rpm] | [mW] | [s] | | [s] |
| PE/PA | INTAREMA 1108 TE | 107 | 48-54 | 750 | 350 | 3 | 10 | 30 |

### Auswertung

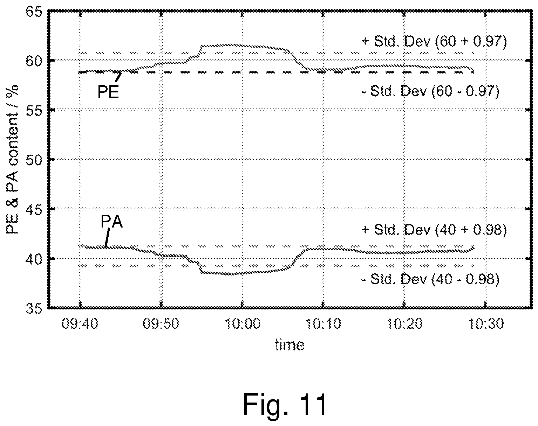

Die vom Detektor 12 erfassten Spektren werden von der Verarbeitungs- und Steuereinheit 40 ausgewertet und der Anteil von PA zu PE wird von der Verarbeitungs- und Steuereinheit 40 dargestellt, siehe dazu Fig. 11.

### Interpretation

Durch unterschiedliche Varianten der verarbeiteten Folien gibt es entsprechende Verschiebungen. So steigt beispielsweise gegen ca. 9:55 der PE-Anteil an. Dies ist aber nicht erwünscht, um eine entsprechende gewünschte Endqualität des Prozessendprodukts zu erzielten, da ein zu geringer PA-Anteil die Eigenschaften des Endproduktes negativ beeinflusst, und darüber hinaus zu viel Verträglichmacher zudosiert wird. Aus diesem Grund kann als Reaktion auf die ermittelten Informationen über das vermessene Material eine Weiche am Ende der Aufbereitungsanlage angeschlossen und das produzierte Granulat im Bereich von 9:55 bis 10:08 ausgeschleust werden.

Durch die oben beschriebene erfindungsgemäße Vorrichtung bzw. das oben beschriebene erfindungsgemäße Verfahren ist somit unter Einsatz von spektroskopischen und/oder spektrometrischen Messvorrichtungen 10, eine effektive Inline-Prozessanalyse, -überwachung und gegebenenfalls -steuerung in Systemen zum Aufbereiten, Bearbeiten und/oder Recyceln von thermoplastischen Kunststoffmaterialien, mit einem Aufnahmebehälter 1, d.h. einem Schneidverdichter, ermöglicht.

Weiters können die von der Auswertungseinheit übermittelten Informationen/Daten auch in der nachfolgenden Prozesskette, wie z.B. beim Zudosierung von Füllstoffen, der Ausschleusung von fertigem Granulat aber auch bei der Zuführung von anderen Polymeren z.B. in den Aufnahmebehälter 1, d.h. den Schneidverdichter, verwendet werden.

## Patentansprüche

1. Verfahren zum Aufbereiten, Bearbeiten oder Recyceln von Materialien, nämlich von thermoplastischen Kunststoffmaterialien,
- wobei das Material in einem Aufnahmebehälter (1), nämlich einem Schneidverdichter, bewegt und gemischt wird, und auch erwärmt, zerkleinert und/oder erweicht wird, wobei das Material im Aufnahmebehälter (1) durchwegs stückig bzw. teilchenförmig und unaufgeschmolzen bleibt, und
- wobei das sich im Inneren des Aufnahmebehälters (1) bewegende stückige bzw. teilchenförmige Material inline spektroskopisch und/oder spektrometrisch analysiert oder gemessen wird, wobei die derart ermittelten Messwerte zur Gewinnung von Informationen über das jeweils vermessene Material, insbesondere von quantitativen und/oder qualitativen Kenngrößen des jeweiligen Materials, herangezogen werden,
wobei zumindest Teile des im Inneren des Aufnahmebehälters (1) befindlichen und dort rotierenden stückigen bzw. teilchenförmigen Materials durch eine physikalische Einwirkung, nämlich durch elektromagnetische Strahlung, angeregt wird und die als Reaktion auf die Einwirkung entstehenden Messsignale, insbesondere charakteristische Spektren der am vermessenen Material gestreuten elektromagnetischen Strahlung, spektroskopisch und/oder spektrometrisch detektiert werden,
wobei die spektroskopische und/oder spektrometrische Vermessung mittels Atomspektroskopie oder Molekülspektroskopie erfolgt,
wobei zur Anregung Licht im Bereich des infraroten, des sichtbaren und/oder des UV-Lichts verwendet wird, im Bereich von 100 nm bis 1400 nm, oder wobei zur Anregung ein Laser verwendet wird, mit einem Wellenlängenbereich von 100 nm bis 1400 nm,
wobei Licht detektiert und analysiert wird, um spezifische quantitative und/oder qualitative Kenngrößen des thermoplastischen Kunststoffmaterials, bzw. Veränderungen dieser Kenngrößen während des Prozesses inline zu erfassen und zur Überwachung und/oder Steuerung des Prozesses heranzuziehen und/oder diese Kenngrößen zur Steuerung der Prozessführung im Aufnahmebehälter (1) zu verwenden,
wobei die das Material anregende elektromagnetische Strahlung auf einen Fokuspunkt (23) fokussiert wird, der im Inneren des Aufnahmebehälters (1) am oder unmittelbar hinter der Behälterwand, in einem Abstand von maximal 10 cm hinter der Behälterwand, liegt,
wobei durch die elektromagnetische Strahlung ein durch eine Messfleck-Querschnittsfläche von 0,1 mm bis 5 mm, und eine Eindringtiefe in das Material von 0,3 µm bis 30 µm, definierter Volumenbereich angeregt wird,
wobei das stückige bzw. teilchenförmige Material im äußeren Bereich des Aufnahmebehälters (1) an der Seitenwand (2) des Aufnahmebehälters (1) eine Bewegungsrichtung in Umfangsrichtung und eine vorwiegend aufwärtsgerichtete Bewegungsrichtung aufweist,
wobei das stückige bzw. teilchenförmige Material radial mit einer Geschwindigkeit von 0,3 m/s bis 45 m/s und in vertikaler Richtung mit einer Geschwindigkeit von 0,1 m/s bis 60 m/s umläuft, sodass das stückige bzw. teilchenförmige Material im äußeren Bereich des Aufnahmebehälters (1), insbesondere an der Seitenwand (2) des Aufnahmebehälters (1), häufig und regelmäßig ausgetauscht wird.

2. Verfahren nach Anspruch 1, **dadurch gekennzeichnet, dass** die Atomspektroskopie oder Molekülspektroskopie mittels Raman-Spektroskopie, NIR-Spektroskopie, UV/VIS-Spektroskopie, Fluoreszenz-Spektroskopie oder Absorptions-Spektroskopie erfolgt.

3. Verfahren nach einem der Ansprüche 1 bis 2, **dadurch gekennzeichnet, dass** zur Anregung Licht im Bereich von 500 nm bis 1000 nm verwendet wird, oder dass ein zur Anregung verwendeter Laser eine Leistung im Bereich von 15 mW bis 5 W, vorzugsweise von 100 mW bis 500 mW aufweist.

4. Verfahren nach einem der Ansprüche 1 bis 3, **dadurch gekennzeichnet, dass** durch die elektromagnetische Strahlung ein durch eine Messfleck-Querschnittsfläche von 1 mm bis 3 mm, und eine Eindringtiefe in das Material von 8 µm bis 15 µm definierter Volumenbereich angeregt wird.

5. Verfahren nach einem der Ansprüche 1 bis 4, **dadurch gekennzeichnet, dass** die das Material anregende physikalische Einwirkung, nämlich die elektromagnetische Strahlung, an einer oder mehreren der folgenden Positionen in das Innere des Aufnahmebehälters (1) bzw. in das Material eingebracht wird:
- unterhalb des Füllniveaus des im Betrieb im Aufnahmebehälter (1) befindlichen Materials bzw. der Materialteilchen,
- in einer Höhe und/oder in einem Abstand zum Boden (3) oder zum Misch- und/oder Zerkleinerungswerkzeug (4), in der/dem die physikalische Einwirkung, nämlich die elektromagnetische Strahlung, ständig unterhalb des verfahrensmäßig vorgegebenen Füllstandes der im Aufnahmebehälter (1) befindlichen bzw. rotierenden Materialteilchen und/oder unterhalb des Niveaus der bei einer Bewegung und/oder Rotation der Materialteilchen ausgebildeten Mischtrombe liegt,
- in Höhe des mittleren Drittelbereichs des verfahrensmäßig vorgegebenen Füllstandes des Materials im Aufnahmebehälter (1) und/oder der Mischtrombe,
- in demjenigen Bereich des Aufnahmebehälters (1), in dem die Dichte der bewegten und/oder rotierenden Materialteilchen am höchsten ist, und/oder
- in demjenigen Bereich des Aufnahmebehälters (1), in dem die bewegten und/oder rotierenden Materialteilchen den höchsten Druck auf die Seitenwand (2) des Aufnahmebehälters (1) ausüben.

6. Verfahren nach einem der Ansprüche 1 bis 5, **dadurch gekennzeichnet,**
**dass** das im Inneren des Aufnahmebehälters (1) befindliche und dort rotierende stückige bzw. teilchenförmige Material, insbesondere einzelne Teilchen des Materials, zu einer Vielzahl an vorgegebenen Zeitpunkten durch eine physikalische Einwirkung, nämlich elektromagnetische Strahlung, angeregt wird und
**dass** der Mittelwert der Informationen über das jeweils vermessene Material, insbesondere über die einzelnen Teilchen, vorzugweise der quantitativen und/oder qualitativen Kenngrößen des jeweiligen Materials, die auf Grundlage ausgewählter, vorzugsweise aller, zu diesen Zeitpunkten ermittelten Messwerte ermittelt wurden, berechnet und zur Verfügung gehalten wird.

7. Verfahren nach einem der Ansprüche 1 bis 6, **dadurch gekennzeichnet,**
**dass** das im Inneren des Aufnahmebehälters (1) befindliche und dort rotierende stückige bzw. teilchenförmige Material während eines vorgegebenen Zeitraums, insbesondere von mehreren Sekunden, kontinuierlich durch eine physikalische Einwirkung, nämlich elektromagnetische Strahlung, angeregt wird und
**dass** für den jeweiligen Zeitraum eine gemeinsame Information über das jeweils vermessene Material, insbesondere eine quantitative und/oder qualitative Kenngröße, auf Grundlage der innerhalb dieses Zeitraums kontinuierlich ermittelten Messwerte berechnet und zur Verfügung gehalten wird.

8. Verfahren nach einem der Ansprüche 1 bis 7, **dadurch gekennzeichnet, dass** die Temperatur im Inneren des Aufnahmebehälters (1) und/oder die Temperatur des Materials gemessen wird und die Temperaturinformation in die Auswertung einbezogen wird und als Indikation für die Korrektur der Informationen über das jeweils vermessene Material, insbesondere der quantitativen und/oder qualitativen Kenngrößen des jeweiligen Materials, dient und/oder die Materialtemperatur erfasst wird und als Vorgabe für die Korrektur der Spektren herangezogen wird.

9. Verfahren nach einem der Ansprüche 1 bis 8, **dadurch gekennzeichnet,**
- **dass** Referenzinformationen, insbesondere quantitative und/oder qualitative Referenzkenngrößen, vorzugsweise Referenzspektren, hinterlegt werden und
- **dass** die für das jeweils vermessene Material ermittelten Informationen, insbesondere die Kenngrößen, vorzugsweise die Spektren, mit den Referenzinformationen, insbesondere den Referenzkenngrößen, vorzugsweise den Referenzspektren, verglichen und die Abweichung von den Referenzinformationen, insbesondere den Referenzkenngrößen, vorzugsweise den Referenzspektren, bestimmt werden, und insbesondere angezeigt und/oder zur Überwachung und/oder Steuerung der Prozessführung im Aufnahmebehälter (1) und/oder der nachfolgenden Prozesskette herangezogen werden.

10. Vorrichtung zur Durchführung eines Verfahrens nach einem der Ansprüche 1 bis 9, mit zumindest einem Aufnahmebehälter (1), nämlich einem Schneidverdichter, mit einer Misch- und/oder Zerkleinerungseinrichtung für das Material sowie mit einer spektroskopischen und/oder spektrometrischen Messvorrichtung (10) zur Analyse des sich im Inneren des Aufnahmebehälters (1) bewegenden stückigen bzw. teilchenförmigen Materials bzw. zur Gewinnung von Informationen über das jeweils vermessene thermoplastische Kunststoffmaterial, insbesondere von quantitativen und/oder qualitativen Kenngrößen des jeweiligen thermoplastischen Kunststoffmaterials, wobei die Vorrichtung aufweist:
- zumindest einen Aufnahmebehälter (1), nämlich einen Schneidverdichter, mit einer Misch- und/oder Zerkleinerungseinrichtung für das Material,
- zumindest eine spektroskopische und/oder spektrometrische Messvorrichtung (10) zur Inline-Vermessung von Teilen des sich im Inneren des Aufnahmebehälters (1) bewegenden stückigen bzw. teilchenförmigen thermoplastischen Kunststoffmaterials, die dazu ausgebildet ist,
- eine physikalische Einwirkung, nämlich elektromagnetische Strahlung, zur Anregung des rotierenden stückigen bzw. teilchenförmigen Materials abzugeben und
- die als Reaktion auf die Einwirkung entstehenden Messsignale, insbesondere charakteristische Spektren der am vermessenen Material gestreuten elektromagnetischen Strahlung, vorzugsweise spektrometrisch, zu detektieren
und
- eine mit der Messvorrichtung (10) in Datenkommunikation stehende Verarbeitungs- und Steuereinheit (40), die dazu ausgebildet ist,
- die Messvorrichtung (10) anzusteuern, die physikalische Einwirkung, nämlich elektromagnetische Strahlung, abzugeben und die entstehenden Messsignale zu detektieren und die derart ermittelten Messwerte zur Verfügung zu halten und
- auf Grundlage der ermittelten Messwerte Informationen über das jeweils vermessene thermoplastische Kunststoffmaterial, insbesondere quantitative und/oder qualitative Kenngrößen des jeweiligen thermoplastischen Kunststoffmaterials, abzuleiten und zur Verfügung zu halten,
wobei der Aufnahmebehälter (1) eine Seitenwand (2) besitzt und im Wesentlichen konisch oder zylindrisch ist oder einen konischen oder zylindrischen Wandabschnitt aufweist, und gegebenenfalls auch eine untere Bodenfläche (3) aufweist,
wobei im Aufnahmebehälter (1) als Misch- und/oder Zerkleinerungseinrichtung zumindest ein, insbesondere um eine vertikale Drehachse (9) drehbares, umlaufendes Misch- und/oder Zerkleinerungswerkzeug (4) zur Bewegung und Mischung, und auch zur Erwärmung, Zerkleinerung und/oder Erweichung, des zu behandelnden stückigen bzw. teilchenförmigen thermoplastischen Kunststoffmaterials angeordnet ist, wobei im Betrieb im Aufnahmebehälter (1) ein Wirbel und/oder eine Mischtrombe, ausbildbar ist,
wobei die Umfangsgeschwindigkeit des Misch- und/oder Zerkleinerungswerkzeugs (4) derart gewählt ist, dass das stückige bzw. teilchenförmige Material im Betrieb radial mit einer Geschwindigkeit von 0,3 m/s bis 45 m/s und in vertikaler Richtung mit einer Geschwindigkeit von 0,1 m/s bis 60 m/s umläuft,
wobei die Verarbeitungs- und Steuereinheit (40) zur spektrometrischen und/oder spektroskopischen Analyse der als Reaktion auf die Einwirkung entstehenden Messsignale, insbesondere von charakteristischen Spektren der am vermessenen thermoplastischen Kunststoffmaterial gestreuten elektromagnetischen Strahlung, ausgebildet ist,
wobei die Messvorrichtung (10)
- zumindest eine, ins Innere des Aufnahmebehälters (1) wirkende bzw. gerichtete Anregungsquelle (11) zur Abgabe der physikalischen Einwirkung, nämlich von elektromagnetischer Strahlung, auf zumindest Teile des im Betrieb im Inneren des Aufnahmebehälters (1) befindlichen und dort rotierenden stückigen bzw. teilchenförmigen Materials sowie
- zumindest einen Detektor (12), insbesondere ein Spektroskop, zur Erfassung der als Reaktion auf die Einwirkung entstehenden Messsignale, insbesondere charakteristischer Spektren der am vermessenen thermoplastischen Kunststoffmaterial gestreuten elektromagnetischen Strahlung, umfasst,
wobei die Messvorrichtung (10) einen Detektor (12) zur Atomspektroskopie oder Molekülspektroskopie umfasst,
wobei die Anregungsquelle (11) dazu ausgebildet ist, Licht im Bereich des infraroten, des sichtbaren und/oder des UV-Lichts, im Bereich von 100 nm bis 1400 nm, abzugeben, oder
wobei die Anregungsquelle (11) ein Laser ist, mit einem Wellenlängenbereich von 100 nm bis 1400 nm oder einer Leistung im Bereich von 15 mW bis 5 W,
wobei eine Linse oder ein Linsensystem (22) zur Fokussierung der elektromagnetischen Strahlung der Anregungsquelle (11) auf einen Fokuspunkt (23) vorgesehen ist,
wobei der durch die physikalische Einwirkung, nämlich die elektromagnetische Strahlung, anregbare Volumenbereich derart eingestellt ist, dass eine Messfleck-Querschnittsfläche von 0,1 mm bis 5 mm und eine Eindringtiefe in das Material von 0,3 µm bis 30 µm definiert ist,
wobei im Aufnahmebehälter (1), nämlich in der Seitenwand (2) des Aufnahmebehälters (1), zumindest eine Messöffnung (20) vorgesehen ist, wobei die Messöffnung (20) derart ausgebildet ist, dass die von der Anregungsquelle (11) abgegebene physikalische Einwirkung, nämlich die abgegebene elektromagnetische Strahlung, auf das Material im Aufnahmebehälter (1) einwirkt und Streulicht aus dem Aufnahmebehälter (1) detektierbar ist,
wobei die zumindest eine Messöffnung (20) in der Seitenwand (2) des Aufnahmebehälters (1) an einer oder mehreren der folgenden Positionen angeordnet ist/sind:
- im Bereich der Höhe des untersten, bodennächsten Misch- und/oder Zerkleinerungswerkzeugs (4), und/oder
- im Bereich des unteren Drittels der Höhe des Aufnahmebehälters (1),
wobei die Verarbeitungs- und Steuereinheit (40) mit einer Prozess-Steuerungseinheit (50) zusammenwirkt und in Datenkommunikation steht, wobei die Prozess-Steuerungseinheit (50) dazu ausgebildet ist, von der Verarbeitungs- und Steuereinheit (40) übermittelte Daten, nämlich Informationen über das jeweils vermessene thermoplastische Kunststoffmaterial, vorzugsweise quantitative und/oder qualitative Kenngrößen des jeweiligen thermoplastischen Kunststoffmaterials, zur Überwachung und/oder Steuerung der Prozessführung im Aufnahmebehälter (1) und/oder der nachfolgenden Prozesskette zu nutzen.

11. Vorrichtung nach Anspruch 10, **dadurch gekennzeichnet, dass** im Aufnahmebehälter (1), insbesondere in einer Seitenwand (2) des Aufnahmebehälters (1), insbesondere im Bereich der Höhe des oder des untersten, bodennächsten Misch- und/oder Zerkleinerungswerkzeugs (4), eine Öffnung (8) ausgebildet ist, durch die das vorbehandelte Kunststoffmaterial aus dem Inneren des Aufnahmebehälters (1) ausbringbar ist, und dass zumindest ein Förderer, insbesondere ein Extruder (5), mit zumindest einer in einem Gehäuse (16) rotierenden, insbesondere plastifizierenden oder agglomerierenden, Schnecke (6), zur Aufnahme des aus der Öffnung (8) austretenden vorbehandelten Materials angeordnet ist, wobei insbesondere vorgesehen ist, dass das Gehäuse (16) eine an seiner Stirnseite oder in seiner Mantelwand liegende Einzugsöffnung (80) für das von der Schnecke (6) zu erfassende Material aufweist, und die Einzugsöffnung (80) mit der Öffnung (8) in Verbindung steht.

12. Vorrichtung nach einem der Ansprüche 10 bis 11, **dadurch gekennzeichnet, dass** die Anregungsquelle (11) und/oder der Detektor (12) und/oder die Verarbeitungs- und Steuereinheit (40) vom Aufnahmebehälter (1) physikalisch beabstandet oder am Aufnahmebehälter (1) vibrationsfrei, insbesondere über faseroptische Systeme und/oder Lichtleiter (14), angekoppelt sind.

13. Vorrichtung nach einem der Ansprüche 10 bis 12, **dadurch gekennzeichnet, dass** der Detektor (12) zur Atomspektroskopie oder Molekülspektroskopie ein Detektor zur Raman-Spektroskopie, NIR-Spektroskopie, UV/VIS-Spektroskopie, Fluoreszenz-Spektroskopie und/oder Absorptions-Spektroskopie ist.

14. Vorrichtung nach einem der Ansprüche 10 bis 13, **dadurch gekennzeichnet, dass** die Anregungsquelle (11) dazu ausgebildet ist, Licht im Bereich von 500 nm bis 1000 nm abzugeben.

15. Vorrichtung nach einem der Ansprüche 10 bis 14, **dadurch gekennzeichnet, dass** die Anregungsquelle (11) ein Laser ist, mit einer Leistung im Bereich von 100 mW bis 500 mW.

16. Vorrichtung nach einem der Ansprüche 10 bis 15, **dadurch gekennzeichnet, dass** die Messöffnung (20) einen Durchmesser von 0,5 mm bis 100 mm hat.

17. Vorrichtung nach einem der Ansprüche 10 bis 16, **dadurch gekennzeichnet, dass** die Messöffnung (20) durch ein Fenster (21) aus, insbesondere für elektromagnetische Strahlung, durchlässigem Material, beispielsweise aus Saphirglas, verschlossen ist.

18. Vorrichtung nach Anspruch 17, **dadurch gekennzeichnet, dass** das Fenster (21) eine Stärke von 1 mm bis 100 mm aufweist.

19. Vorrichtung nach einem der Ansprüche 17 oder 18, **dadurch gekennzeichnet, dass** die Flächen des Fensters (21) planeben und zueinander parallel ausgerichtet sind oder dass die dem Aufnahmebehälter (1) zugewendete inneren Fläche des Fensters (21) konkav dem Radius des Aufnahmebehälters (1) angepasst ist und die dem Aufnahmebehälter (1) abgewendete äußere Fläche des Fensters (21) parallel konkav zur inneren Fläche ausgebildet ist.

20. Vorrichtung nach einem der Ansprüche 10 bis 19, **dadurch gekennzeichnet, dass** die zumindest eine Messöffnung (20) in der Seitenwand (2) des Aufnahmebehälters (1) an einer oder mehreren der folgenden Positionen angeordnet ist/sind: etwas oberhalb oder unterhalb von der Höhe des untersten, bodennächsten Misch- und/oder Zerkleinerungswerkzeugs (4), vorzugsweise beim engsten Abstand zwischen dem äußersten Punkt des Misch- und/oder Zerkleinerungswerkzeugs (4) und der Seitenwand (2).

21. Vorrichtung nach einem der Ansprüche 10 bis 19, **dadurch gekennzeichnet, dass** die zumindest eine Messöffnung (20), insbesondere in der Seitenwand (2) des Aufnahmebehälters (1), an einer oder mehreren der folgenden Positionen angeordnet ist/sind:
- unterhalb des Füllniveaus des im Betrieb im Aufnahmebehälter (1) befindlichen Materials bzw. der Materialteilchen,
- in einer Höhe und/oder in einem Abstand zum Boden (3) oder zum Misch- und/oder Zerkleinerungswerkzeug (4), in der/dem die physikalische Einwirkung, nämlich die elektromagnetische Strahlung, ständig unterhalb des verfahrensmäßig vorgegebenen Füllstandes der im Aufnahmebehälter (1) befindlichen bzw. rotierenden Materialteilchen und/oder des Niveaus der bei einer Bewegung und/oder Rotation der Materialteilchen ausgebildeten Mischtrombe liegt,
- in Höhe des mittleren Drittelbereichs des verfahrensmäßig vorgegebenen Füllstandes des Materials im Aufnahmebehälter (1) und/oder der Mischtrombe,
- in demjenigen Bereich des Aufnahmebehälters (1), in dem die Dichte der bewegten und/oder rotierenden Materialteilchen am höchsten ist, und/oder
- in demjenigen Bereich des Aufnahmebehälters (1), in dem die bewegten und/oder rotierenden Materialteilchen den höchsten Druck auf die Seitenwand (2) des Aufnahmebehälters (1) ausüben.

22. Vorrichtung nach einem der Ansprüche 10 bis 21, **dadurch gekennzeichnet, dass** die Messfleck-Querschnittsfläche 1 mm bis 3 mm, und die Eindringtiefe in das Material 8 µm bis 15 µm ist.

23. Vorrichtung nach einem der Ansprüche 10 bis 22, **dadurch gekennzeichnet, dass** der Fokuspunkt (23) am oder unmittelbar hinter dem Fenster (21), vorzugsweise in einem Abstand von maximal 10 cm hinter dem Fenster (21), ausgebildet ist.

24. Vorrichtung nach einem der Ansprüche 10 bis 23, **dadurch gekennzeichnet, dass** die Prozess-Steuerungseinheit (50) dazu ausgebildet ist, auf Grundlage der von der Verarbeitungs- und Steuereinheit (40) übermittelten Daten
- eine Zudosierung von Füllstoffen in den Aufnahmebehälter (1) vorzunehmen und/oder
- eine Zuführung von Materialien, insbesondere Polymeren, in den Aufnahmebehälter (1) und/oder in eine an den Aufnahmebehälter (1) angeschlossene Austragungsvorrichtung vorzunehmen und/oder
- verarbeitete Materialien, insbesondere Granulat, aus dem Aufnahmebehälter (1) mittels der an den Aufnahmebehälter (1) angeschlossenen Austragungsvorrichtung auszuschleusen.

25. Vorrichtung nach einem der Ansprüche 10 bis 24, **dadurch gekennzeichnet, dass** zumindest eine der Verarbeitungs- und Steuereinheit (40) vorgeschaltete Temperaturmesseinrichtung (30) vorgesehen ist, wobei die Temperaturmesseinrichtung (30) dazu ausgebildet ist, die Temperatur im Inneren des Aufnahmebehälters (1) und/oder die Temperatur des Materials zu messen und an die Verarbeitungs- und Steuereinheit (40) zu übermitteln, und wobei die Verarbeitungs- und Steuereinheit (40) dazu ausgebildet ist, die von der zumindest einen Temperaturmesseinrichtung (30) ermittelten Messwerte für eine Korrektur des Temperatureinflusses auf die für das jeweils vermessene Material ermittelten Informationen, insbesondere der temperaturabhängigen charakteristischen Spektren der am vermessenen Material gestreuten elektromagnetischen Strahlung, heranzuziehen und die derart korrigierten Informationen, insbesondere Spektren, zur Verfügung zu halten.

26. Vorrichtung nach Anspruch 25, **dadurch gekennzeichnet, dass** die Temperaturmesseinrichtung (30) im Aufnahmebehälter (1) auf gleicher Höhe, insbesondere an gleicher Position, wie die zumindest eine Messöffnung (20) angeordnet ist.

27. Vorrichtung nach einem der Ansprüche 10 bis 26, **dadurch gekennzeichnet, dass** die Verarbeitungs- und Steuereinheit (40) dazu ausgebildet ist,
- die Messvorrichtung (10), insbesondere die Anregungsquelle (11), zu einer Vielzahl an vorgegebenen Zeitpunkten anzusteuern, wiederholt eine physikalische Einwirkung, nämlich elektromagnetische Strahlung, abzugeben und
- den Mittelwert der Informationen über das jeweils vermessene thermoplastische Kunststoffmaterial, insbesondere über die einzelnen vermessenen Teilchen, vorzugsweise der quantitativen und/oder qualitativen Kenngrößen des jeweiligen thermoplastischen Kunststoffmaterials, die auf Grundlage ausgewählter, vorzugsweise aller, zu diesen Zeitpunkten von der Messvorrichtung (10), insbesondere dem Detektor (12), ermittelten Messwerte ermittelt wurden, zu berechnen und zur Verfügung zu halten.

28. Vorrichtung nach einem der Ansprüche 10 bis 27, **dadurch gekennzeichnet, dass** die Verarbeitungs- und Steuereinheit (40) dazu ausgebildet ist,
- die Messvorrichtung (10), nämlich die Anregungsquelle (11), anzusteuern, eine physikalische Einwirkung, nämlich elektromagnetische Strahlung, während eines vorgegebenen Zeitraums, insbesondere von mehreren Sekunden, kontinuierlich abzugeben und
- für den jeweiligen Zeitraum eine gemeinsame Information über das jeweils vermessene Material, insbesondere eine quantitative und/oder qualitative Kenngröße, auf Grundlage der von der Messvorrichtung (10), nämlich dem Detektor (12), innerhalb dieses Zeitraums kontinuierlich ermittelten Messwerte zu berechnen und zur Verfügung zu halten.

29. Vorrichtung nach einem der Ansprüche 10 bis 28, **dadurch gekennzeichnet,**
**dass** die Verarbeitungs- und Steuereinheit (40) einen Speicher umfasst, wobei in dem Speicher Referenzinformationen, insbesondere quantitative und/oder qualitative Referenzkenngrößen, vorzugsweise Referenzspektren, hinterlegt sind und
**dass** die Verarbeitungs- und Steuereinheit (40) dazu ausgebildet ist, die für das jeweils vermessene thermoplastische Kunststoffmaterial ermittelten Informationen, insbesondere die Kenngrößen, vorzugsweise die Spektren, mit den Referenzinformationen, insbesondere den Referenzkenngrößen, vorzugsweise den Referenzspektren, zu vergleichen und die Abweichung von den Referenzinformationen, insbesondere den Referenzkenngrößen, vorzugsweise den Referenzspektren, zu bestimmen, und insbesondere an die Prozess-Steuerungseinheit und/oder eine Anzeigeeinheit weiterzuleiten.

## Claims

1. Method for the treating, processing or recycling of materials, specifically of thermoplastic materials,
- wherein the material is moved and mixed in a receiving container (1), specifically a cutting compactor, and is also heated, comminuted and/or softened, wherein the material in the receiving container (1) remains in chunks or in particulate form and non-melted, and
- wherein the chunky or particulate material moving in the interior of the receiving container (1) is subjected to spectroscopic and/or spectrometric inline analysis or measurement, wherein the measurement values determined in this manner are used to obtain information regarding the respectively measured material, particularly quantitative and/or qualitative characteristic variables of the respective material,
wherein at least some of the chunky or particulate material, located in the interior of the receiving container (1) and rotating there, is excited by a physical action, specifically by electromagnetic radiation, and the measurement signals resulting as a reaction to the excitation, in particular characteristic spectra of the electromagnetic radiation scattered by the measured material, are detected spectroscopically and/or spectrometrically,
wherein the spectroscopic and/or spectrometric measurement is carried out by means of atomic spectroscopy and/or molecular spectroscopy,
wherein for the excitation, light is used in the infrared, visible and/or ultraviolet range, in the range from 100 nm to 1400 nm, or wherein for the excitation a laser is used with a wavelength range from 100 nm to 1400 nm,
wherein light is detected and analysed in order to detect inline specific quantitative and/or qualitative characteristics of the thermoplastic material, or changes in these characteristic variables during the process, and to draw upon them to monitor and/or control the process and/or to use these characteristic variables to control the process management in the receiving container (1),
wherein the electromagnetic radiation exciting the material is focused onto a focal point (23) which lies in the interior of the receiving container (1) on or immediately behind the container wall, at a distance of maximum 10 cm behind the container wall,
wherein by means of the electromagnetic radiation a volume region is excited which is defined by a measuring spot cross-sectional area of 0.1 to 5 mm and a penetration depth into the material of 0.3 µm to 30 µm,
wherein the chunky or particulate material in the outer region of the receiving container (1) at the side wall (2) of the receiving container (1) has a direction of movement in circumferential direction and a predominantly upwardly-directed direction of movement,
wherein the chunky or particulate material circulates radially at a speed of 0.3 m/s to 45 m/s and in vertical direction at a speed of 0.1 m/s to 60 m/s, such that the chunky or particulate material in the outer scope of the receiving container (1), particularly at the side wall (2) of the receiving container (1) is frequently and regularly exchanged.

2. Method according to claim 1, **characterised in that** the atomic spectroscopy or molecular spectroscopy is carried out by means of Raman spectroscopy, NIR spectroscopy, UV/Vis spectroscopy, fluorescence spectroscopy or absorption spectroscopy.

3. Method according to any of claims 1 to 2, **characterised in that** for the excitation light is used in the range from 500 nm to 1000 nm, or that a laser used for the excitation has a power in the range from 15 mW to 5 W, preferably from 100 mW to 500 mW.

4. Method according to any of claims 1 to 3, **characterised in that** by means of the electromagnetic radiation a volume region is excited which is defined by a measuring spot cross-sectional area of 1 mm to 3 mm and a penetration depth into the material of 8 µm to 15 µm.

5. Method according to any of claims 1 to 4, **characterised in that** the physical action, specifically the electromagnetic radiation, exciting the material is introduced into the interior of the receiving container (1) or into the material at one or several of the following positions:
- below the filling level of the material or the material particulate located in the receiving container (1) during operation,
- at a level and/or at a distance from the base (3) or from the mixing and/or comminuting tool (4), at which the physical action, specifically the electromagnetic radiation, lies permanently below the procedurally specified filling level of the material particles located or rotating in the receiving container (1) and/or below the level of the mixing vortex formed by a movement and/or rotation of the material particles,
- at a level of the middle third of the procedurally specified filling level of the material in the receiving container (1) and/or the mixing vortex,
- in the region of the receiving container (1) in which the density of the moved and/or rotating material particles is highest, and/or
- in the region of the receiving container (1) in which the moved and/or rotating material particles exerts the highest pressure on the side wall (2) of the receiving container (1).

6. Method according to any of claims 1 to 5, **characterised in**
**that** the chunky or particulate material located in the interior of the receiving container (1) and rotating there, in particular individual particles of the material, is excited by a physical action, specifically electromagnetic radiation, at a multiplicity of predetermined time points, and
**that** the average value of the information regarding the respectively measured material, particularly regarding the individual particles, preferably of the quantitative and/or qualitative characteristic variables of the respective material, which were determined on the basis of selected, preferably all, measurement values determined at these time points, is calculated and kept available.

7. Method according to any of claims 1 to 6, **characterised in**
**that** the chunky or particulate material, located in the interior of the receiving container (1) and rotating there, is excited continuously by a physical action, specifically electromagnetic radiation, during a predetermined time period, particularly of several seconds, and
**that** for the respective time period a common item of information regarding the respectively measured material, particularly a quantitative and/or qualitative characteristic variable, is calculated on the basis of the measurement values determined continuously within this time period, and is kept available.

8. Method according to any of claims 1 to 7, **characterised in that** the temperature in the interior of the receiving container (1) and/or the temperature of the material is measured and the temperature information is included in the evaluation and serves as an indication for the correction of the items of information regarding the respectively measured material, in particular of the quantitative and/or qualitative characteristics of the respective material, and/or the material temperature is detected and used as a reference for the correction of the spectra.

9. Method according to any of claims 1 to 8, **characterised in**
- **that** items of reference information, in particular quantitative and/or qualitative reference characteristic variables, preferably reference spectra, are stored, and
- **that** the items of information determined for the respectively measured material, in particular the characteristic variables, preferably the spectra, are compared with the items of reference information, in particular the reference characteristics, preferably the reference spectra, and the deviation from the items of reference information, in particular from the reference characteristic variables, preferably from the reference spectra, is specified, and is in particular displayed and/or used for monitoring and/or controlling the process management in the receiving container (1) and/or the subsequent process chain.

10. Device for carrying out a method according to any of claims 1 to 9, having at least one receiving container (1), specifically a cutting compactor, having a mixing and/or comminuting apparatus for the material as well as having a spectroscopic and/or spectrometric measuring device (10) for analyzing the chunky or particulate material moving in the interior of the receiving container (1) or for obtaining information regarding the respectively measured thermoplastic material, in particular quantitative and/or qualitative characteristic variables of the respective thermoplastic material, wherein the device has:
- at least one receiving container (1), specifically a cutting compactor having a mixing and/or comminuting apparatus for the material,
- at least one spectroscopic and/or spectrometric measuring device (10), for the inline measurement of some of the chunky or particulate thermoplastic material moving in the interior of the receiving container (1), which is configured
- to emit a physical action, specifically electromagnetic radiation, for exciting the rotating chunky or particulate material and
- to detect, preferably in a spectrometric manner, the measurement signals, in particular characteristic spectra of the electromagnetic radiation scattered on the measured material, as a reaction to the action, and
- a processing and control unit (40) being in data communication with the measuring device (10), which is configured
- to control the measuring device (10) to emit the physical action, specifically electromagnetic radiation, and to detect the resulting measurement signals and to keep available the measurement values determined in this manner, and
- on the basis of the determined measurement values to derive information regarding the respectively measured thermoplastic material, particularly quantitative and/or qualitative characteristic variables of the respective thermoplastic material, and to keep it available,
wherein the receiving container (1) has a side wall (2) and is substantially conical or cylindrical or has a conical or cylindrical wall portion, and where appropriate has also a lower base surface (3),
wherein in the receiving container (1) is arranged as a mixing and/or comminuting device at least one rotary, particularly rotatable about a vertical rotational axis (9), mixing and/or comminuting tool (4) for moving and mixing, and also for heating, comminuting and/or softening, the chunky or particulate thermoplastic material to be treated, wherein during operation a swirl and/or a mixing vortex can be formed in the receiving container (1),
wherein the circumferential speed of the mixing and/or comminuting tool (4) is selected such that the chunky or particulate material circulates during operation radially at a speed of 0.3 m/s to 45 m/s and in vertical direction at a speed of 0.1 m/s to 60 m/s,
wherein the processing and control unit (40) is designed for the spectrometric and/or spectroscopic analysis of the measurement signals, in particular of characteristic spectra of the electromagnetic radiation scattered on the measured thermoplastic material, resulting as a reaction to the action,
wherein the measuring device (10) includes
- at least one excitation source (11), acting or aligned into the interior of the receiving container (1) for emitting the physical action, specifically electromagnetic radiation, onto at least some of the chunky or particulate material located during operation in the interior of the receiving container (1) and rotating there, as well as
- at least one detector (12), particularly a spectroscope, for detecting the measurement signals resulting as a reaction to the impact, in particular characteristic spectra of the electromagnetic radiation scattered on the measured thermoplastic material,
wherein the measuring device (10) includes a detector (12) for atomic spectroscopy or molecular spectroscopy,
wherein the excitation source (11) is configured to emit light in the range of infrared, visible and/or ultraviolet light in the range from 100 nm to 1400 nm;
or
wherein the excitation source (11) is a laser with a wavelength range of 100 nm to 1400 nm or a power in the range of 15 mW to 5 W.
wherein a lens or a lens system (22) is provided for focusing the electromagnetic radiation of the excitation source (11) onto a focal point (23),
wherein the volume scope which can be excited by the physical action, specifically the electromagnetic radiation, is adjusted such that a measuring spot cross-sectional area of 0.1 mm to 5 mm and a penetration depth into the material of 0.3 µm to 30 µm is defined,
wherein in the receiving container (1), specifically in the side wall (2) of the receiving container (1), is provided at least one measuring opening (20), wherein the measuring opening (20) is designed such that the physical action emitted by the excitation source (11), specifically the emitted electromagnetic radiation, acts on the material in the receiving container (1) and scattered light from the receiving container (1) can be detected,
wherein the at least one measuring opening (20) in the side wall (2) of the receiving container (1) is/are arranged at one or several of the following positions:
- in the region of the level of the lowermost mixing and/or comminuting tool (4) closest to the base, and/or
- in the region of the lower third of the height of the receiving container (1),
wherein the processing and control unit (40) interacts with, and is in data communication with, a process-control unit (50), wherein the process-control unit (50) is configured to use data transmitted by the processing and control unit (40), specifically information regarding the respectively measured thermoplastic material, preferably quantitative and/or qualitative characteristic variables of the respective thermoplastic material, for monitoring and/or controlling the process management in the receiving container (1) and/or to use the subsequent process chain.

11. Device according to claim 10, **characterised in that** in the receiving container (1), in particular in a side wall (2) of the receiving container (1), in particular in the region of the level of the or the lowermost mixing and/or comminuting tool (4) closest to the base, is formed an opening (8) through which the pretreated plastic material can be removed from the interior of the receiving container (1), and that at least one conveyor, in particular an extruder (5), having at least one, in particular plasticising or agglomerating, screw (6) rotating in a housing (16), is arranged for receiving the pretreated material emerging out of the opening (8), wherein in particular it is provided that the housing (16) has an infeed opening (80) located on its end face or in its jacket wall for the material to be captured by the screw (6), and the infeed opening (80) is connected to the opening (8).

12. Device according to any of claims 10 to 11, **characterised in that** the excitation source (11) and/or the detector (12) and/or the processing and control unit (40) are physically spaced from the receiving container (1) or are coupled to the receiving container (1) in a vibration-free manner, particularly via fibre-optic systems and/or light guides (14).

13. Device according to any of claims 10 to 12, **characterised in that** the detector (12) for atomic spectroscopy or molecular spectroscopy is a detector for Raman spectroscopy, NIR spectroscopy, UV/Vis spectroscopy, fluorescence spectroscopy and/or absorption spectroscopy.

14. Device according to any of claims 10 to 13, **characterised in that** the excitation source (11) is configured to emit light in the range from 500 nm to 1000 nm.

15. Device according to any of claims 10 to 14, **characterised in that** the excitation source (11) is a laser with a power in the range from 100 mW to 500 mW.

16. Device according to any of claims 10 to 15, **characterised in that** the measuring opening (20) has a diameter of 0.5 mm to 100 mm.

17. Device according to any of claims 10 to 16, **characterised in that** the measuring opening (20) is closed by a window (21) of material, for example sapphire glass, which is permeable, particularly for electromagnetic radiation.

18. Device according to claim 17, **characterised in that** the window (21) has a thickness of 1 mm to 100 mm.

19. Device according to any of claims 17 or 18, **characterised in that** the surfaces of the window (21) are planar and aligned parallel to one another or that the inner surface of the window (21) facing the receiving container (1) is adapted in a concave manner to the radius of the receiving container (1) and the outer surface of the window (21) facing away from the receiving container is designed so as to be concave and parallel to the inner surface.

20. Device according to any of claims 10 to 19, **characterised in that** the at least one measuring opening (20) in the side wall (2) of the receiving container (1) is/are arranged at one or several of the following positions:
slightly below or above the height of the lowermost mixing and comminuting tool (4) closest to the base, preferably at the closest spacing between the outermost point of the mixing and/or commuting tool (4) and the side wall (2).

21. Device according to any of claims 10 to 19, **characterised in that** the at least one measuring opening (20), particularly in the side wall (2) of the receiving container (1), is/are arranged at one or several of the following positions:
- below the filling level of the material or the material particulate located in the receiving container (1) during operation,
- at a level and/or at a distance from the base (3) or from the mixing and/or commuting tool (4), in which the physical action, specifically the electromagnetic radiation, is situated permanently below the procedurally prescribed filling level of the material particles located or rotating in the receiving container (1) and/or below the level of the mixing vortex formed in the case of a movement and/or rotation of the material particles,
- at a level of the middle third of the procedurally specified filling level of the material in the receiving container (1) and/or the mixing vortex,
- in the region of the receiving container (1) in which the density of the moved and/or rotating material particles is highest, and/or
- in the region of the receiving container (1) in which the moved and/or rotating material particles exerts the highest pressure on the side wall (2) of the receiving container (1).

22. Device according to any of claims 10 to 21, **characterised in that** the measuring spot cross-sectional area is 1 mm to 3 mm, and the penetration depth into the material is 8 µm to 15 µm.

23. Device according to any of claims 10 to 22, **characterised in that** the focal point (23) is formed on or immediately behind the window (21), preferably at a distance of maximum 10 cm behind the window (21).

24. Device according to any of claims 10 to 23, **characterised in that** the process-control unit (50) is configured, on the basis of the data transmitted by the processing and control unit (40),
- to make a metered addition of filling materials into the receiving container (1) and/or
- to undertake a feeding of materials, in particular polymers, into the receiving container (1) and/or into a discharge device attached to the receiving container (1) and/or
- to expel processed materials, in particular granulate, out of the receiving container (1) by means of the discharge device attached to the receiving container (1).

25. Device according to any of claims 10 to 24, **characterised in that** at least one temperature measuring device (30) is provided upstream of the processing and control unit (40), wherein the temperature measuring device (30) is configured to measure the temperature in the interior of the receiving container (1) and/or the temperature of the material and to transmit it to the processing and control unit (40), and
wherein the processing and control unit (40) is configured to use the measurement values determined by the at least one temperature measuring device (30) to correct the temperature influence on the information determined for the respectively measured material, in particular the temperature-dependent characteristic spectra of the electromagnetic radiation scattered on the measured material, and to keep available the information, particularly spectra, which have been corrected in this manner.

26. Device according to claim 25, **characterised in that** the temperature measuring device (30) is arranged in the receiving container (1) at the same level, particularly at the same position, as the at least one measuring opening (20).

27. Device according to any of claims 10 to 26, **characterised in that** the processing and control unit (40) is configured
- to trigger the measuring device (10), in particular the excitation source (11), to emit a physical action, specifically electromagnetic radiation, repeatedly at a multiplicity of predetermined time points, and
- to calculate the average value of the items of information regarding the respectively measured thermoplastic material, particularly regarding the individual measured particles, preferably of the quantitative and/or qualitative characteristic variables of the respective thermoplastic material which were determined on the basis of selected, preferably all, measurement values determined at these time points by the measuring device (10), particularly the detector (12), and to keep them available.

28. Device according to any of claims 10 to 27, **characterised in that** the processing and control unit (40) is configured
- to trigger the measuring device (10), specifically the excitation source (11) to emit a physical action, specifically electromagnetic radiation, continuously during a predetermined time period, particularly of several seconds, and
- to calculate for the respective time period a common item of information regarding the respectively measured material, in particular a quantitative and/or qualitative characteristic variable, on the basis of the measurement values determined continuously during this time period by the measuring device (10), specifically the detector (12), and to keep it available.

29. Device according to any of claims 10 to 28, **characterised in**
**that** the processing and control unit (40) includes a memory, wherein in the memory are stored items of reference information, in particular quantitative and/or qualitative reference characteristic variables, preferably reference spectra, and
**that** the processing and control unit (40) is configured to compare the items of information determined for the respectively measured thermoplastic material, in particular the characteristic variables, preferably the spectra, with the items of reference information, in particular the reference characteristic variables, preferably the reference spectra, and to specify the deviation from the items of reference information, in particular the reference characteristic variables, preferably the reference spectra, and in particular to forward them to the process control unit and/or a display unit.

## Revendications

1. Procédé destiné à préparer, à traiter ou à recycler des matériaux, en particulier des matériaux plastiques thermoplastiques,
- dans lequel le matériau bouge et est mélangé dans un récipient d'accueil (1), en particulier un compresseur de coupe, et est également chauffé, concassé et/ou ramolli, dans lequel le matériau reste toujours sous forme de morceaux voire de particules et sous-forme non fondue dans le récipient d'accueil (1), et
- dans lequel le matériau sous forme de morceaux voire de particule bougeant à l'intérieur du récipient d'accueil (1) est analysé ou mesuré en interne par spectroscopie et/ou spectrométrie, dans lequel les valeurs de mesure ainsi déterminées sont utilisées pour la collecte d'informations concernant le matériau mesuré respectif, en particulier de grandeurs caractéristiques quantitatives et /ou qualitatives du matériau respectif,
dans lequel au moins une partie du matériau sous forme de morceaux voire de particules se trouvant et tournant à l'intérieur du récipient d'accueil (1) est excité par une action physique, en particulier par un rayonnement électromagnétique, et les signaux de mesure survenant en réaction à l'action, en particulier des spectres caractéristiques du rayonnement électromagnétique, dispersé au niveau du matériau mesuré, sont détectés de préférence par spectroscopie et/ou spectrométrie,
dans lequel la mesure par spectroscopie et/ou spectrométrie est effectuée au moyen d'une spectroscopie atomique ou d'une spectroscopie moléculaire,
dans lequel une lumière dans la plage de la lumière infrarouge, visible et/ou UV est utilisée pour l'excitation, dans la plage de 100 nm à 1400 nm, ou dans lequel un laser est utilisé pour l'excitation, avec une plage de longueur d'onde de 100 nm à 1400 nm,
dans lequel de la lumière est détectée et analysée afin de recueillir des paramètres quantitatifs et/ou qualitatifs spécifiques du matériau plastique thermoplastique ou des modifications de ces paramètres au cours du processus en interne, et de les utiliser pour surveiller et/ou commander le processus concerné et/ou d'utiliser ces paramètres pour commander la gestion de processus dans le récipient d'accueil (1),
dans lequel le rayonnement électromagnétique excitant le matériau est focalisé sur un point de focalisation (23) qui se trouve à l'intérieur du récipient d'accueil (1) sur ou directement derrière la paroi de récipient, à une distance maximale de 10 cm derrière la paroi de récipient,
dans lequel par l'intermédiaire du rayonnement électromagnétique, une plage volumique définie par une surface de section transversale de point de mesure de 0,1 mm à 5 mm et une profondeur de pénétration dans le matériau de 0,3 µm à 30 µm est excitée,
dans lequel le matériau sous forme de morceaux voire de particules dans la région externe du récipient d'accueil (1) sur la paroi latérale (2) du récipient d'accueil (1) présente une direction de déplacement dans la direction circonférentielle et une direction de déplacement principalement dirigée vers le haut,
dans lequel le matériau sous forme de morceaux voire de particules tourne radialement à une vitesse de 0,3 m/s à 45 m/s et dans une direction verticale à une vitesse de 0,1 m/s à 60 m/s, de sorte que le matériau sous forme de morceaux voire de particules dans l'étendue externe du récipient d'accueil (1), en particulier sur la paroi latérale (2) du récipient d'accueil (1), est remplacé fréquemment et régulièrement.

2. Procédé selon la revendication 1, **caractérisé en ce que** la spectroscopie atomique ou la spectroscopie moléculaire est réalisée au moyen de la spectroscopie Raman, de la spectroscopie NIR, de la spectroscopie UV/VIS, de la spectroscopie de fluorescence ou de la spectroscopie d'absorption.

3. Procédé selon l'une quelconque des revendications 1 à 2, **caractérisé en ce que** pour
qu'une lumière d'excitation dans la plage de 500 nm à 1000 nm est utilisée, ou **en ce qu'**un laser utilisé pour une excitation présente une puissance dans la plage de 15 mW à 5 W, de préférence de 100 mW à 500 mW.

4. Procédé selon l'une quelconque des revendications 1 à 3, **caractérisé en ce qu'**une plage volumique définie par une surface de section transversale de point de mesure de 1 mm à 3 mm et une profondeur de pénétration dans le matériau de 8 µm à 15 µm est excitée par le rayonnement électromagnétique.

5. Procédé selon l'une quelconque des revendications 1 à 4, **caractérisé en ce que** l'action physique excitant le matériau, en particulier le rayonnement électromagnétique, est introduit à l'intérieur du récipient (1) ou dans le matériau dans une ou plusieurs des positions suivantes :
- en dessous du niveau de remplissage du matériau ou des particules de matériau dans le récipient d'accueil (1) pendant le fonctionnement,
- à une hauteur et/ou à une distance du sol (3) ou de l'outil de mélange et/ou de concassage (4) à laquelle l'action physique, en particulier le rayonnement électromagnétique, est constamment inférieur au niveau de remplissage spécifié par le procédé des particules de matériau tournant dans le récipient d'accueil (1) et/ou en dessous du niveau du tourbillon de mélange formé lors du déplacement et/ou de la rotation des particules de matériau,
- à la hauteur du tiers médian du niveau de remplissage du matériau spécifié par le procéde du matériau dans le récipient d'accueil (1) et/ou le tourbillon de mélange,
- dans la zone du récipient d'accueil (1) dans laquelle la densité des particules de matériau en mouvement et/ou en rotation est la plus élevée, et/ou
- dans cette zone du récipient d'accueil (1) dans laquelle les particules de matériau en mouvement et/ou en rotation exercent la plus grande pression sur la paroi latérale (2) du récipient d'accueil (1).

6. Procédé selon l'une quelconque des revendications 1 à 5, **caractérisé en ce que**
le matériau sous forme de morceaux voire de particules se trouvant à l'intérieur du récipient d'accueil (1) et y tournant, en particulier des particules individuelles du matériau, est excité à un grand nombre d'instants prédéterminés par une action physique, en particulier un rayonnement électromagnétique, et
la valeur moyenne des informations sur le matériau mesuré respectif, en particulier sur les particules individuelles, de préférence les paramètres quantitatifs et/ou qualitatifs du matériau respectif, qui ont été déterminées sur la base de valeurs de mesure sélectionnées, de préférence toutes, déterminées à ces instants, est calculé et mis à disposition.

7. Procédé selon l'une quelconque des revendications 1 à 6, **caractérisé**
**en ce que** le matériau sous forme de morceaux voire de particules situé à l'intérieur du récipient (1) et y bougeant est excité en permanence par une action physique, en particulier un rayonnement électromagnétique, pendant une période de temps prédéterminée, en particulier de quelques secondes, et
**en ce que** pour la période de temps respective, des informations communes sur le matériau mesuré respectif, en particulier un paramètre quantitatif et/ou qualitatif, sont calculées et mises à disposition sur la base des valeurs de mesure déterminées en continu pendant cette période de temps.

8. Procédé selon l'une quelconque des revendications 1 à 7, **caractérisé en ce que** la température à l'intérieur du récipient (1) et/ou la température du matériau est mesurée et l'information de température est incluse dans l'évaluation et sert d'indication pour la correction de l'information concernant le matériau mesuré respectif, en particulier les paramètres quantitatifs et/ou qualitatifs du matériau respectif, et/ou la température du matériau est détectée et utilisée comme spécification pour la correction des spectres.

9. Procédé selon l'une quelconque des revendications 1 à 8, **caractérisé en ce que**
- des informations de référence, en particulier des paramètres de référence quantitatifs et/ou qualitatifs, de préférence des spectres de référence, sont stockées et
- les informations déterminées pour le matériau mesuré respectif, en particulier les paramètres, de préférence les spectres, sont comparées aux informations de référence, en particulier les paramètres de référence, de préférence les spectres de référence, et l'écart par rapport aux informations de référence, en particulier les paramètres de référence, de préférence les spectres de référence, est déterminé et en particulier affiché et/ou utilisé pour surveiller et/ou commander la gestion de processus dans le récipient d'accueil (1) et/ou la chaîne de processus suivante.

10. Dispositif pour la mise en œuvre d'un procédé selon l'une quelconque des revendications 1 à 9, avec au moins un récipient d'accueil (1), en particulier un compacteur de coupe, avec un dispositif de mélange et/ou concassage du matériau et avec un dispositif de mesure (10) par spectroscopie et/ou spectrométrie pour analyser le matériau sous forme de morceaux voire de particules bougeant à l'intérieur du récipient (1) ou pour la collecte d'informations sur le matériau plastique thermoplastique mesuré respectif, en particulier des paramètres quantitatifs et/ou qualitatifs du matériau plastique thermoplastique respectif, dans lequel le dispositif présente :
- au moins un récipient d'accueil (1), en particulier un compacteur de coupe, avec un dispositif de mélange et/ou de concassage du matériau,
- au moins un dispositif de mesure (10) par spectroscopie et/ou spectrométrie pour une mesure en interne de parties du matériau plastique thermoplastique sous forme de morceaux voire de particule bougeant à l'intérieur du récipient (1), qui est conçu pour
- délivrer une action physique, en particulier un rayonnement électromagnétique, pour exciter le matériau sous forme de morceaux voire de particules en rotation, et
- détecter, de préférence par spectrométrie, les signaux de mesure survenant en réaction à l'action, en particulier des spectres caractéristiques du rayonnement électromagnétique dispersé sur le matériau mesuré, et
- une unité de traitement et de commande (40) qui est en communication de données avec le dispositif de mesure (10), qui est formée pour
- commander le dispositif de mesure (10), pour délivrer l'action physique, en particulier le rayonnement électromagnétique, et pour détecter les signaux de mesure résultants et pour mettre les valeurs de mesure ainsi déterminées à disposition, et
- sur la base des valeurs de mesure déterminées, dériver et mettre à disposition des informations sur le matériau plastique thermoplastique respectif mesuré, en particulier des paramètres quantitatifs et/ou qualitatifs du matériau plastique thermoplastique respectif,
dans lequel le récipient d'accueil (1) possède une paroi latérale (2) et est essentiellement conique ou cylindrique ou présente une section de paroi conique ou cylindrique, et présente éventuellement une surface de fond inférieure (3),
dans lequel dans le récipient d'accueil (1), en tant que dispositif de mélange et/ou de concassage, est agencé au moins un outil de mélange et/ou de concassage (4) continu, en particulier pouvant tourner autour d'un axe de rotation vertical (9) pour déplacer et mélanger, ainsi que pour chauffer, concasser et/ou ramollir le matériau plastique thermoplastique sous forme de morceaux voire de particules à traiter, dans lequel un vortex et/ou un tourbillon de mélange peut se former dans le récipient d'accueil (1) en fonctionnement,
dans lequel la vitesse périphérique de l'outil de mélange et/ou de concassage (4) est choisie de sorte que le matériau sous forme de morceaux voire de particules circule en fonctionnement radialement à une vitesse de 0,3 m/s à 45 m/s et dans une direction verticale à une vitesse de 0,1 m/s à 60 m/s,
dans lequel l'unité de traitement et de commande (40) est conçue pour une analyse par spectrométrie et/ou spectroscopie des signaux de mesure survenant en réaction à l'action, en particulier de spectres caractéristiques du rayonnement électromagnétique diffusé sur le matériau plastique thermoplastique mesuré,
dans lequel le dispositif de mesure (10) comprend
- au moins une source d'excitation (11) agissant ou dirigée vers l'intérieur du récipient d'accueil (1) pour délivrer l'action physique, en particulier un rayonnement électromagnétique, sur au moins des parties des matériaux sous forme de morceaux voire de particules se trouvant et tournant à l'intérieur du récipient d'accueil (1) en fonctionnement, ainsi que
- au moins un détecteur (12), en particulier un spectroscope, pour détecter les signaux de mesure survenant en réaction à l'action, en particulier des spectres caractéristiques du rayonnement électromagnétique diffusé sur le matériau plastique thermoplastique mesuré,
dans lequel le dispositif de mesure (10) comprend un détecteur (12) pour une spectroscopie atomique ou une spectroscopie moléculaire,
dans lequel la source d'excitation (11) est conçue pour émettre de la lumière dans la plage de la lumière infrarouge, visible et/ou UV, dans la plage de 100 nm à 1400 nm ;
ou
dans lequel la source d'excitation (11) est un laser avec une plage de longueur d'onde de 100 nm à 1400 nm ou une puissance dans la plage de 15 mW à 5 W,
dans lequel une lentille ou un système de lentilles (22) est prévu(e) pour focaliser le rayonnement électromagnétique de la source d'excitation (11) sur un point de focalisation (23),
dans lequel la plage volumique qui peut être excitée par l'action physique, en particulier le rayonnement électromagnétique, est ajustée de telle manière qu'une surface de section transversale de point de mesure de 0,1 mm à 5 mm et une profondeur de pénétration dans le matériau de 0,3 µm à 30 µm est définie,
dans lequel au moins une ouverture de mesure (20) est prévue dans le récipient d'accueil (1), en particulier dans la paroi latérale (2) du récipient d'accueil (1), dans lequel l'ouverture de mesure (20) est conçue de telle sorte que l'action physique délivrée en sortie par la source d'excitation (11), en particulier le rayonnement électromagnétique délivré en sortie, agit sur le matériau dans le récipient d'accueil (1), et de la lumière diffusée provenant du récipient d'accueil (1) peut être détectée,
dans lequel la au moins une ouverture de mesure (20) dans la paroi latérale (2) du récipient d'accueil (1) est/sont agencée(s) dans une ou plusieurs des positions suivantes :
- dans la zone de la hauteur de l'outil de mélange et/ou de concassage le plus bas et le plus proche du sol (4), et/ou
- dans la zone du tiers inférieur de la hauteur du récipient d'accueil (1),
dans lequel l'unité de traitement et de commande (40) interagit et est en communication de données avec une unité de commande de processus (50), dans lequel l'unité de commande de processus (50) est conçue pour utiliser des données transmises par l'unité de traitement et de commande (40), en particulier des informations sur le matériau plastique thermoplastique mesuré respectif, de préférence des paramètres quantitatifs et/ou qualitatifs du matériau plastique thermoplastique respectif, afin de surveiller et/ou commander la réalisation de processus dans le récipient d'accueil (1) et/ou la chaîne de processus suivante.

11. Dispositif selon la revendication 10, **caractérisé en ce que** dans le récipient d'accueil (1), en particulier dans une paroi latérale (2) du récipient d'accueil (1), en particulier dans la zone de la hauteur de l'outil de mélange et/ou de concassage le plus bas et le plus proche du sol (4) est formée une ouverture (8), à travers laquelle le matériau plastique prétraité peut être évacué de l'intérieur du récipient d'accueil (1), et **en ce qu'**au moins un convoyeur, en particulier une extrudeuse (5), avec au moins une vis (6) tournant dans un logement (16), en particulier de plastification ou d'agglomération, est agencé pour recevoir le matériau prétraité sortant de l'ouverture (8), dans lequel il est en particulier prévu que le logement (16) présente une ouverture d'alimentation (80) située sur sa face avant ou dans sa paroi d'envelopper pour le matériau à détecter provenant de la vis (6), et l'ouverture d'alimentation (80) communique avec l'ouverture (8).

12. Dispositif selon l'une quelconque des revendications 10 à 11, **caractérisé en ce que** la source d'excitation (11) et/ou le détecteur (12) et/ou l'unité de traitement et de commande (40) sont physiquement espacés du récipient d'accueil (1) ou sont couplés au récipient d'accueil (1) sans vibration, en particulier via des systèmes à fibres optiques et/ou des guides de lumière (14).

13. Dispositif selon l'une quelconque des revendications 10 à 12, **caractérisé en ce que** le détecteur (12) pour une spectroscopie atomique ou une spectroscopie moléculaire est un détecteur pour la spectroscopie Raman, la spectroscopie NIR, la spectroscopie UV/VIS, la spectroscopie de fluorescence ou la spectroscopie d'absorption.

14. Dispositif selon l'une quelconque des revendications 10 à 13, **caractérisé en ce que** la
source d'excitation (11) est conçue pour émettre de la lumière dans la plage de 500 nm à 1000 nm.

15. Dispositif selon l'une quelconque des revendications 10 à 14, **caractérisé en ce que** la source d'excitation (11) est un laser d'une puissance dans la plage de 100 mW à 500 mW.

16. Dispositif selon l'une quelconque des revendications 10 à 15, **caractérisé en ce que** l'ouverture de mesure (20) présente un diamètre de 0,5 mm à 100 mm.

17. Dispositif selon l'une quelconque des revendications 10 à 16, **caractérisé en ce que** l'ouverture de mesure (20) est fermée par une fenêtre (21) en matériau transparent, en particulier pour un rayonnement électromagnétique, par exemple en verre saphir.

18. Dispositif selon la revendication 17, **caractérisé en ce que** la fenêtre (21) présente une épaisseur de 1 mm à 100 mm.

19. Dispositif selon l'une quelconque des revendications 17 ou 18, **caractérisé en ce que** les surfaces de la fenêtre (21) sont conçues planes et alignées parallèlement entre elles ou **en ce que** la surface intérieure de la fenêtre (21) tournée vers le récipient d'accueil (1) est adaptée concave avec le rayon du récipient d'accueil (1) et la surface extérieure de la fenêtre (21) tournée à l'opposé du récipient d'accueil (1) est réalisée concave parallèlement à la surface intérieure.

20. Dispositif selon l'une quelconque des revendications 10 à 19, **caractérisé en ce que** la au moins une ouverture de mesure (20) dans la paroi latérale (2) du récipient d'accueil (1) est/sont agencée(s) dans une ou plusieurs des positions suivantes :
légèrement au-dessus ou au-dessous de la hauteur de l'outil de mélange et/ou de concassage le plus bas et le plus proche du sol (4), de préférence à la distance la plus étroite entre le point le plus à l'extérieur de l'outil de mélange et/ou de concassage (4) et la paroi latérale (2).

21. Dispositif selon l'une quelconque des revendications 10 à 19, **caractérisé en ce que** la au moins une ouverture de mesure (20), en particulier dans la paroi latérale (2) du récipient d'accueil (1), est/sont agencée(s) dans une ou plusieurs des positions suivantes :
- en dessous du niveau de remplissage du matériau ou des particules de matériau se trouvant dans le récipient d'accueil (1) en fonctionnement,
- à une hauteur et/ou à une distance du sol (3) ou de l'outil de mélange et/ou de concassage (4) à laquelle l'action physique, en particulier le rayonnement électromagnétique, est constamment inférieur au niveau de remplissage spécifié par le procédé dans le récipient (1) situé ou bougeant les particules de matériau et/ou le niveau du tourbillon de mélange formé lors du mouvement et/ou de la rotation des particules de matériau,
- à la hauteur du tiers médian du niveau de remplissage du matériau spécifié par le procéde du matériau dans le récipient d'accueil (1) et/ou le tourbillon de mélange,
- dans la zone du récipient d'accueil (1) dans laquelle la densité des particules de matériau en mouvement et/ou en rotation est la plus élevée, et/ou
- dans cette zone du récipient d'accueil (1) dans laquelle les particules de matériau en mouvement et/ou en rotation exercent la plus grande pression sur la paroi latérale (2) du récipient d'accueil (1).

22. Dispositif selon l'une quelconque des revendications 10 à 21, **caractérisé en ce que** la surface de section transversale de point de mesure est de 1 mm à 3 mm, et la profondeur de pénétration dans le matériau est de 8 µm à 15 µm.

23. Dispositif selon l'une quelconque des revendications 10 à 22, **caractérisé en ce que** le point de focalisation (23) est formé sur ou directement derrière la fenêtre (21), de préférence à une distance maximale de 10 cm derrière la fenêtre (21).

24. Dispositif selon l'une quelconque des revendications 10 à 23, **caractérisé en ce que** l'unité de commande de processus (50) est conçue pour, sur la base des données transmises par l'unité de traitement et de commande (40)
- effectuer un dosage de charges dans le récipient (1) et/ou
- effectuer une alimentation en matériaux, en particulier en polymères, dans le récipient d'accueil (1) et/ou dans un dispositif d'évacuation relié au récipient d'accueil (1) et/ou
- pour éjecter les matériaux traités, en particulier les granulés, à partir du récipient d'accueil (1) au moyen du dispositif d'évacuation relié au récipient d'accueil (1).

25. Dispositif selon l'une quelconque des revendications 10 à 24, **caractérisé en ce qu'**au moins un dispositif de mesure de température (30) est prévu en amont de l'unité de traitement et de commande (40), dans lequel le dispositif de mesure de température (30) est conçu pour mesurer la température à l'intérieur du récipient d'accueil (1) et/ou la température du matériau et la transmettre à l'unité de traitement et de commande (40), et
dans lequel l'unité de traitement et de commande (40) est conçue pour mettre à disposition les valeurs de mesure déterminées par le au moins un dispositif de mesure de température (30) pour une correction de l'influence de la température sur les informations déterminées pour le matériau mesuré respectif, en particulier les spectres caractéristiques dépendant de la température du rayonnement électromagnétique diffusé sur le matériau mesuré, et les informations ainsi corrigées, en particulier les spectres.

26. Dispositif selon la revendication 25, **caractérisé en ce que** le dispositif de mesure de température (30) est agencé dans le récipient d'accueil (1) à la même hauteur, en particulier dans la même position, que la au moins une ouverture de mesure (20).

27. Dispositif selon l'une quelconque des revendications 10 à 26, **caractérisé en ce que** l'unité de traitement et de commande (40) est conçue pour
- commander le dispositif de mesure (10), en particulier la source d'excitation (11), à un grand nombre d'instants prédéterminés, pour émettre de manière répétée une action physique, en particulier un rayonnement électromagnétique, et
- pour calculer et mettre à disposition la valeur moyenne des informations sur le matériau plastique thermoplastique mesuré respectif, en particulier sur les particules individuelles mesurées, de préférence les paramètres quantitatifs et/ou qualitatifs du matériau plastique thermoplastique respectif, qui ont été déterminés sur la base de valeurs de mesure sélectionnées, de préférence toutes, à ces instants par le dispositif de mesure (10), en particulier le détecteur (12).

28. Dispositif selon l'une quelconque des revendications 10 à 27, **caractérisé en ce que** l'unité de traitement et de commande (40) est conçue pour
- commander le dispositif de mesure (10), en particulier la source d'excitation (11), pour émettre en continu une action physique, en particulier un rayonnement électromagnétique, pendant une période de temps prédéterminée, en particulier plusieurs secondes, et
- calculer et mettre à disposition, pour la période de temps respective, des informations communes sur le matériau mesuré respectif, en particulier un paramètre quantitatif et/ou qualitatif, sur la base des valeurs de mesure déterminées en continu par le dispositif de mesure (10), en particulier le détecteur (12), dans cette période de temps.

29. Dispositif selon l'une quelconque des revendications 10 à 28, **caractérisé**
**en ce que** l'unité de traitement et de commande (40) comprend une mémoire, dans lequel des informations de référence, en particulier des paramètres de référence quantitatifs et/ou qualitatifs, de préférence des spectres de référence, sont stockées dans la mémoire, et
**en ce que** l'unité de traitement et de commande (40) est conçue pour comparer les informations déterminées pour le matériau plastique thermoplastique mesuré respectif, en particulier les paramètres, de préférence les spectres, avec les informations de référence, en particulier les paramètres de référence, de préférence les spectres de référence, et pour estimer l'écart par rapport aux informations de référence, en particulier les paramètres de référence, de préférence les spectres de référence, et en particulier pour le transmettre à l'unité de commande de processus et/ou à une unité d'affichage.
